(19) Europäisches Patentamt
European Patent Office
Office européen des brevets

(11) Veröffentlichungsnummer: **0 235 089 B1**

# (12) EUROPÄISCHE PATENTSCHRIFT

(45) Veröffentlichungstag der Patentschrift: **21.11.91**

(51) Int. Cl.5: **C07C 275/54,** C07C 273/18, A01N 47/34, C07C 217/84

(21) Anmeldenummer: **87810100.5**

(22) Anmeldetag: **23.02.87**

Teilanmeldung 91101254.0 eingereicht am 23/02/87.

Die Akte enthält technische Angaben, die nach dem Eingang der Anmeldung eingereicht wurden und die nicht in dieser Patentschrift enthalten sind.

## (54) N-Benzoyl-N'-phenylharnstoffe, ihre Herstellung und Verwendung bei der Kontrolle von Schädlingen.

(30) Priorität: **28.02.86 CH 808/86**
**19.11.86 CH 4617/86**
**24.12.86 CH 5238/86**

(43) Veröffentlichungstag der Anmeldung:
**02.09.87 Patentblatt 87/36**

(45) Bekanntmachung des Hinweises auf die Patenterteilung:
**21.11.91 Patentblatt 91/47**

(84) Benannte Vertragsstaaten:
**AT BE CH DE ES FR GB GR IT LI NL**

(56) Entgegenhaltungen:
**WO-A-86/07355**
**US-A- 4 518 804**

(73) Patentinhaber: **CIBA-GEIGY AG**
**Klybeckstrasse 141**
**CH-4002 Basel(CH)**

(72) Erfinder: **Drabek, Jozef, Dr.**
**Benkenstrasse 12**
**CH-4104 Oberwil(CH)**

EP 0 235 089 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte N-Benzoyl-N'-2,5-dihalogen-4-halogenalkoxyphenyl-harnstoffe, Verfahren und Zwischenprodukte zu ihrer Herstellung und ihre Verwendung in der Schädlingsbekämpfung.

Die erfindungsgemässen Verbindungen entsprechen der Formel I

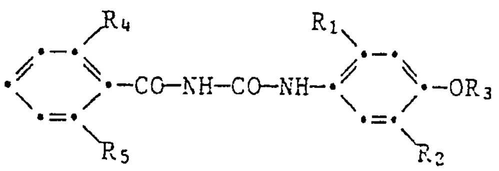

(I),

worin $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen.

Bei den als Substituenten in Betracht kommenden Halogenen handelt es sich sowohl um Fluor und Chlor als auch um Brom und Jod, wobei Fluor, Chlor und Brom bevorzugt sind.

N-Benzoyl-N'-fluoralkoxyphenyl-harnstoffe mit insektizder Wirkung sind aus der Literatur wie beispielweise dem US-Patent 4 518 804 bekannt. Weiter wurden solche Substanzen in der älteren europäischen Patentanmeldung EP-A-0 226 642 publiziert. Diese Verbindungen befriedigen jedoch hinsichtlich ihrer Wirkung nicht immer. Es besteht deshalb weiterhin ein Bedürfnis nach Verbindungen dieses Typs mit verbesserten Eigenschaften.

Unter den Verbindungen der Formel I stehen diejenigen im Vordergrund, in denen $R_1$ und $R_2$ je Fluor, Chlor oder Brom bedeuten, wobei $R_1$ und $R_2$ nicht gleichzeitig Chlor sind, $R_3$ für $-CF_2-CHF-CF_3$, $R_4$ für Wasserstoff, Fluor oder Chlor und $R_5$ für Fluor, Chlor oder Methoxy stehen.

Dabei sind diejenigen Verbindungen der Formel I bevorzugt, in denen $R_1$ Fluor und $R_2$ Fluor, Chlor oder Brom oder $R_1$ Chlor und $R_2$ Fluor bedeuten, $R_3$ für $-CF_2-CHF-CF_3$, $R_4$ für Wasserstoff, Fluor oder Chlor und $R_5$ für Fluor, Chlor oder Methoxy stehen.

Besondere Erwähnung verdienen dabei diejenigen Verbindungen der Formel I, in denen $R_1$ Fluor und $R_2$ Fluor, Chlor oder Brom bedeuten, $R_3$ für $CF_2CHFCF_3$, $R_4$ für Wasserstoff oder Fluor und $R_5$ für Fluor, Chlor oder Methoxy stehen.

Vor allem sind diejenigen Verbindungen der Formel I hervorzuheben, in denen $R_1$ Fluor und $R_2$ Fluor, Chlor oder Brom bedeuten, $R_3$ für $CF_2CHFCF_3$, $R_4$ für Wasserstoff oder Fluor und $R_5$ für Fluor, Chlor oder Methoxy stehen.

Beispiele von Verbindungen der Formel I sind u. a.:

| $R_1$ | $R_2$ | $R_3$ | $R_4$ | $R_5$ |
|---|---|---|---|---|
| F | Cl | $CF_2CHFCF_3$ | F | F |
| F | Cl | $CF_2CHFCF_3$ | $SCH_3$ | F |
| Cl | F | $CF_2CHFCF_3$ | $OCH_3$ | $OCH_3$ |

Die erfindungsgemässen Verbindungen können nach an sich bekannten Verfahren hergestellt werden. Solche Verfahren sind u.a. in den DE-OS 21 23 236, 26 01 780 und 32 40 975 beschrieben. So können die Verbindungen der Formel I z.B. erhalten werden, indem man

a) ein Anilin der Formel II

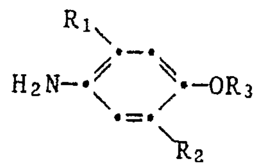

(II)

mit einem Benzoylisocyanat der Formel III

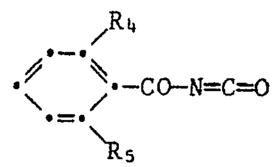

(III),

oder

b) ein Isocyanat der Formel IV

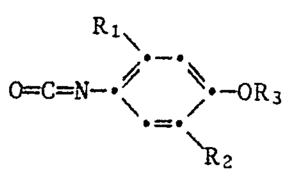

(IV)

mit einem Benzamid der Formel V

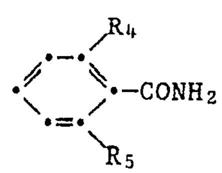

(V),

oder

c) ein Anilin der Formel II mit einem Urethan der Formel VI

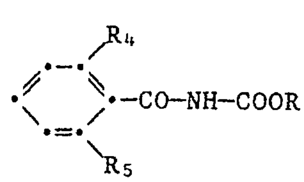

(VI)

umsetzt. In den obigen Formeln II bis VI haben die Reste $R_1$, $R_2$, $R_3$, $R_4$ und $R_5$ die für Formel I angegebene Bedeutung und R steht für einen $C_1$-$C_8$-Alkylrest, der gegebenenfalls mit Halogen, vorzugsweise Chlor, substituiert sein kann.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis +200° C, vorzugsweise zwischen 0 und 100° C, z.B.

bei Raumtemperatur, gegebenenfalls in Gegenwart einer organischen Base, z.B. Triäthylamin, durchgeführt. Die Durchführung von Verfahren b) erfolgt bei einer Temperatur von 0 bis 150 °C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d.h. für die Umsetzung der Urethane der Formel VI mit einem Anilin der Formel III, werden Temperaturen zwischen etwa 60 °C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III und V sind bekannt und können analog bekannten Verfahren hergestellt werden.

Bei den Ausgangsstoffen der Formel II handelt es sich um neuartige Verbindungen, die ebenfalls einen Gegenstand der vorliegenden Erfindung bilden. Die Verbindungen der Formel II können in an sich bekannter Weise z.B. dadurch hergestellt werden, dass man entsprechend substituierte Nitrobenzole der Formel VII

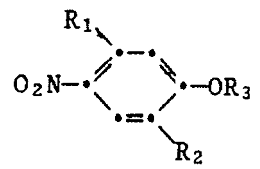

(VII)

in Analogie zu dem in J. Org. Chem. 29 (1964), 1, aufgezeigten Verfahren hydriert (vgl. auch die dort zitierte Literatur). Aber auch durch chemische Reduktion (z.B. mittels Sn-(II)-Chlorid/HCl) der entsprechenden Nitroverbindungen der Formel VII sind Aniline der Formel II zugänglich (Vgl. Houben Weyl, "Methoden d. org. Chemie" 11/1, 422). Eine weitere Herstellungsmöglichkeit für die Aniline der Formel II besteht darin, dass man ein gegebenenfalls acyliertes 2,5-Dihalogen-4-hydroxyanilin haloalkyliert und dann gegebenenfalls die Acylgruppe entfernt, z.B. durch saure Hydrolyse.

Die Nitroverbindungen der Formel VII sind ebenfalls neu und bilden einen Gegenstand der vorliegenden Erfindung. Sie sind z.B. herstellbar durch Haloalkylierung eines 2,5-Dihalogen-4-Nitrophenols (vgl. französische Patentschrift 2,005,876) oder durch Umsetzen von 2,5-Dihalogen-4-fluor-nitrobenzol mit einem Halogenalkanol in alkalischer Lösung und Dimethylsulfoxid als Lösungsmittel (vgl. "The Chemistry of the Hydroxyl Group", Seiten 83-124; Interscience Publishers Inc., New York, 1971).

Zu Benzoylisocyanaten gemäss Formel III kann man unter anderem wie folgt gelangen (vgl. J. Agr. Food Chem. 21, 348 und 993; 1973):

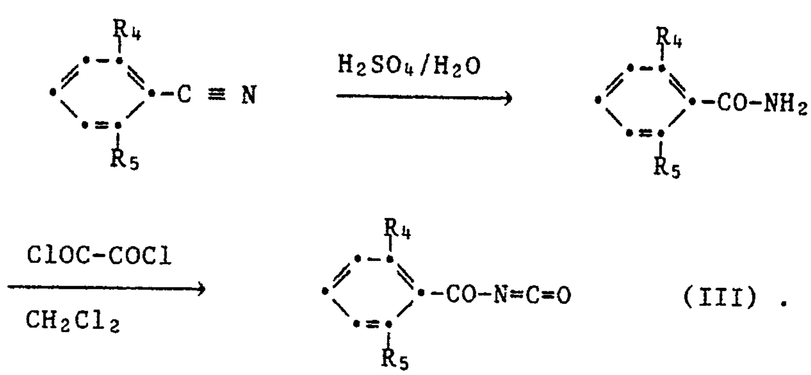


(III) .

Die 4-Haloalkoxy-phenylisocyanate der Formel IV lassen sich z.B. durch Phosgenierung der Aniline der Formel II nach allgemein üblichen Verfahren herstellen. Die weiterhin als Ausgangsstoffe zu verwendenden Benzamide der Formel V sind bekannt (vgl. z.B. Beilstein "Handbuch der organischen Chemie" Bd. 9, S. 336).

Urethane der Formel VI können in an sich bekannter Weise erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure Cl-COOR.

Ueberraschenderweise wurde gefunden, dass die erfindungsgemässen Verbindungen bei günstiger

Warmblüter- und Pflanzenverträglichkeit wertvolle Wirkstoffe in der Schädlingsbekämpfung sind. So eignen sich die Verbindungen der Formel I z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus- und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und wiesen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99%, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung

Die im Anschluss aufgeführten Herstellungsvorschriften betreffen teilweise Substanzen, welche nicht erfindungsgemäss beansprucht werden. Diese Vorschriften werden jedoch als Illustration der erfindungsgemässen Verfahren zur Gewährleistung der Ausführbarkeit der Erfindung in Analogieverfahren beibehalten.

1.1. Zwischenprodukte

1.1.1 Nitrobenzole

2-Fluor-4-(2,2,2-trifluoräthoxy)-5-chlor-nitrobenzol

22,5 g 88%iges Kaliumhydroxid werden in 120 ml Dimethylsulfoxid suspendiert. Zu dieser Suspension werden tropfenweise unter Rühren 40 g Trifluoräthanol gegeben. Die entstandene Lösung lässt man unter Rühren bei Raumtemperatur in eine Lösung von 96,8 g 2,4-Difluor-5-chlor-nitrobenzol in 150 ml Dimethylsulfoxid tropfen. Nach beendeter Zugabe wird noch 2 Stunden bei Raumtemperatur weitergerührt. Alsdann wird das Reaktionsgemisch eingeengt, das Rohprodukt in Methylenchlorid gelöst, die Lösung mit Wasser

gewaschen und getrocknet und schliesslich das Lösungsmittel abdesilliert. Die Reinigung erfolgt chromatographisch an einer Kieselgelsäule mit Hexan/Aether 10:1 als Laufmittel. Nach Abdestillation des Lösungsmittels liegt die Titelverbindung der Formel

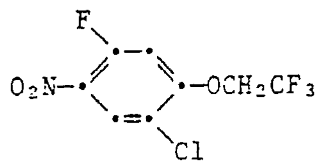

in Form gelber Kristalle vor; Smp. 54-55 ° C.

1.1.2. Aniline

2-Fluor-4-(2,2,2-trifluoräthoxy)-5-chloranilin

21,3 g des obigen Nitrobenzols werden in 220 ml Tetrahydrofuran gelöst und bei Raumtemperatur während 19 Stunden in Gegenwart von 4 g Raney-Nickel hydriert ($H_2$-Aufnahme: 5,26 l). Das Reaktionsgemisch wird filtriert, das Lösungsmittel abdestilliert und der Rückstand destilliert. Die Titelverbindung der Formel

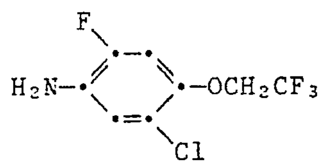

weist einen Siedepunkt von 166 ° C/0,08 Torr und einen Schmelzpunkt von 40-41 ° C auf.

1.1.2.1. 2-Fluor-(4-1,1,2,3,3,3-hexafluorpropoxy)-5-bromanilin

28 g 2-Brom-4-amino-5-fluorphenol und 1,9 ml Triäthylamin werden in 150 ml Dimethylformamid vorgelegt worauf 22,5 g Hexafluorpropylen bei Raumtemperatur eingeleitet werden. Dabei steigt die Temperatur auf 50 ° C. Anschliessend wird das Lösungsmittel im Vakuum abdestilliert, der Rückstand in Aether aufgenommen und die ätherische Lösung zweimal mit verdünnter Natronlauge und zweimal mit Wasser extrahiert. Der über Natriumsulfat getrocknete ätherische Auszug wird fraktioniert destilliert. Die Titelverbindung der Formel

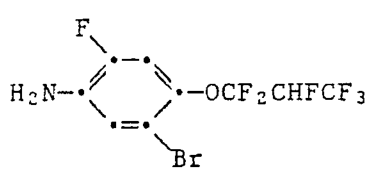

(Verb. Nr. 1.1.2.1.)

liegt in Form einer hellgelben Flüssigkeit vor; Sdp. 76-81 ° C/ 0,001 Torr.

Auf analoge Weise werden die folgenden Verbindungen hergestellt:

| Verb. Nr. | $R_1$ | $R_2$ | $R_3$ | phys. Daten |
|---|---|---|---|---|
| 1.1.2.2. | F | Cl | $CF_2CHFCF_3$ | Sdp. 74-76 ° C/0,01 Torr |
| 1.1.2.3. | F | F | $CF_2CHFCF_3$ | Sdp. 56-58 ° C/0,15 Torr |

1.2. Endprodukt

N-(2,6-Difluorbenzoyl)-N'-[2-fluor-4-(2,2,2-trifluoräthoxy)-5-chlor-phenyl]-harnstoff

4 g 2-Fluor-4-(2,2,2-trifluoräthoxy)-5-chlor-anilin, gelöst in 50 ml trockenem Toluol, werden bei Raumtemperatur mit 3,0 g 2,6-Difluorbenzoylisocyanat, gelöst in 10 ml trockenem Toluol, versetzt und 10 Stunden lang gerührt. Anschliessend werden etwa 75 % des Lösungsmittels am Rotationsverdampfer entfernt. Der gebildete Niederschlag wird abgenutscht, mit wenig kaltem Toluol und Hexan gewaschen und im Vakuum getrocknet. Die Titelverbindung der Formel

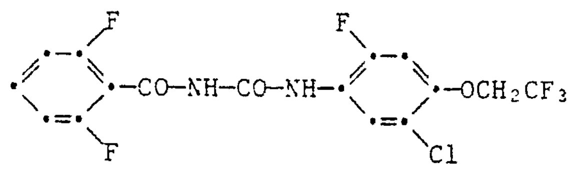

liegt als weisses, kristallines Pulver vor; Smp. 201-202 °C.

Auf analoge Weise werden die folgenden Verbindungen hergestellt:

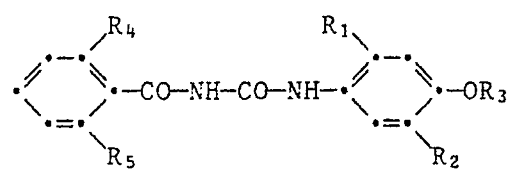

| Verb. Nr. | $R_1$ | $R_2$ | $R_3$ | $R_4$ | $R_5$ | Smp. °C |
|---|---|---|---|---|---|---|
| 1.2.1 | F | Cl | $CF_2CHFCF_3$ | F | F | 163-164 |
| 1.2.2 | F | Cl | $CF_2CHFCF_3$ | H | Cl | 138-140 |
| 1.2.3 | F | Br | $CF_2CHFCF_3$ | F | F | 159-161 |
| 1.2.4 | F | Br | $CF_2CHFCF_3$ | H | Cl | 142-143 |
| 1.2.5 | F | Br | $CF_2CHFCF_3$ | H | F | 139-140 |
| 1.2.6 | F | Br | $CF_2CHFCF_3$ | F | Cl | 190-192 |
| 1.2.7 | F | Br | $CF_2CHFCF_3$ | F | $OCH_3$ | 172-174 |
| 1.2.8 | F | F | $CF_2CHFCF_3$ | F | F | 165-166 |
| 1.2.9 | F | F | $CF_2CHFCF_3$ | H | Cl | 169-170 |
| 1.2.10 | F | F | $CF_2CHFCF_3$ | H | F | 163-164 |
| 1.2.11 | F | F | $CF_2CHFCF_3$ | F | Cl | 191-193 |
| 1.2.12 | F | Cl | $CF_2CHFCF_3$ | H | F | 135-136 |
| 1.2.13 | F | Cl | $CF_2CHFCF_3$ | F | Cl | 198-199 |
| 1.2.14 | F | Cl | $CF_2CHFCF_3$ | Cl | Cl | 184-185 |
| 1.2.15 | F | Cl | $CF_2CHFCF_3$ | F | $OCH_3$ | 172-173 |
| 1.2.16 | F | Cl | $CF_2CHFCF_3$ | H | $OCH_3$ | 162-163,5 |
| 1.2.17 | F | Cl | $CF_2CHFCF_3$ | $OCH_3$ | $OCH_3$ | 175-177 |
| 1.2.18 | F | Br | $CF_2CHFCF_3$ | Cl | Cl | 181-182 |
| 1.2.19 | F | Br | $CF_2CHFCF_3$ | H | $OCH_3$ | 159-161 |
| 1.2.20 | F | Br | $CF_2CHFCF_3$ | $OCH_3$ | $OCH_3$ | 184-185 |
| 1.2.21 | F | F | $CF_2CHFCF_3$ | Cl | Cl | |
| 1.2.22 | F | F | $CF_2CHFCF_3$ | F | $OCH_3$ | |
| 1.2.23 | F | F | $CF_2CHFCF_3$ | H | $OCH_3$ | |
| 1.2.24 | F | F | $CF_2CHFCF_3$ | $OCH_3$ | $OCH_3$ | |

Beispiel 2: Formulierungen von Wirkstoffen der Formel I gemäss Herstellungsbeispiel 1.2

(% = Gewichtsprozent)

| 2.1 Emulsions-Konzentrate | a) | b) |
|---|---|---|
| Wirkstoff gemäss dem Herstellungsbeispiel 1.2 | 10 % | 25 % |
| Ca-Dodecylbenzolsulfonat | - | 5 % |
| Ricinusöl-polyäthylenglykoläther (36 Mol AeO) | 25 % | 5 % |
| Tributylphenol-polyäthylenglykoläther (30 Mol AeO) | - | - |
| Cyclohexanon | - | - |
| Butanol | 15 % | - |
| Xylolgemisch | - | 65 % |
| Essigester | 50% | - |

Aus solchen Konzentraten können durch Verdünnen mit Wasser Emulsionen jeder gewünschten Konzentration hergestellt werden.

| 2.2 Lösungen | a) | b) |
|---|---|---|
| Wirkstoff gemäss dem Herstellungsbeispiel 1.2 | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190° C) | - | 74 % |

Die Lösungen sind zur Anwendung in Form kleinster Tropfen geeignet.

| 2.3 Granulate | a) | b) |
|---|---|---|
| Wirkstoff gemäss dem Herstellungsbeispiel 1.2 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Extruder Granulat | |
|---|---|
| Wirkstoff gemäss dem Herstellungsbeispiel 1.2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5 Umhüllungs-Granulat | |
|---|---|
| Wirkstoff gemäss dem Herstellungsbeispiel 1.2 | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin

gleichmässig aufgetragen. Auf diese Weise erhält man staubfreie Umhüllungs-Granulate.

| 2.6 Stäubemittel | a) | b) | c) | d) |
|---|---|---|---|---|
| Wirkstoff gemäss dem Herstellungsbeispiel 1.2 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| 2.7 Spritzpulver | a) | b) | c) |
|---|---|---|---|
| Wirkstoff gemäss dem Herstellungsbeispiel 1.2 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

| 2.8 Suspensions-Konzentrat | |
|---|---|
| Wirkstoff gemäss dem Herstellungsbeispiel 1.2 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologische Prüfungen

3.1 Wirkung gegen Musca domestica

Je 50 g frisch zubereitetes CSMA-Nährsubstrat für Maden werden in einen Becher eingewogen. Von einer 1 gew.%igen acetonischen Lösung des Wirkstoffes werden 5 ml auf das im Becher befindliche Nährsubstrat pipettiert. Nach dem Durchmischen des Substrates lässt man das Aceton mindestens 20 Stunden lang verdampfen.

Dann werden 25 eintägige Maden von Musca domestica in den das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in einem mit einem Siebdeckel verschlossenen Gefäss deponiert.

Die ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen gemäss Beispiel 1.2 zeigen gute Wirkung im obigen Test.

3.2 Wirkung gegen Lucilia sericata

Zu 9 ml eines Zuchtmediums wird bei 50 °C 1 ml einer 0.5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss Beispiel 1.2 zeigen in diesem Test gute Wirkung gegen Lucilia sericata.

3.3 Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss Beispiel 1.2 zeigen gute Wirkung in diesem Test.

3.4 Insektizide Frassgift-Wirkung

Baumwollpflanzen (ca. 20 cm hoch) werden mit einer wässrigen Wirkstoffemulsion (erhalten aus einem 10%igen emulgierbaren Konzentrat) besprüht, wobei die Wirkstoffemulsion 100 ppm der zu prüfenden Verbindung enthält.

Nach dem Antrocknen des Belages werden die Baumwollpflanzen je mit Spodoptera littoralis- und Heliothis virescens-Larven im dritten larvalen Stadium besetzt. Der Versuch wird bei 24 °C und 60 % relativer Luftfeuchtigkeit durchgeführt. In Abständen von jeweils 24 Stunden werden Mortalität sowie Entwicklungs- und Häutungsstörungen der angesetzten Larven bestimmt.

Verbindungen gemäss Beispiel 1.2 zeigen gute Wirkung in diesem Test.

3.5 Wirkung auf Spodoptera littoralis und Heliothis virescens (Larven und Eier)

Es werden drei in Töpfen gezogene Baumwollpflanzen von ca. 15-20 cm Höhe mit einer sprühfähigen flüssigen Zubereitung des zu prüfenden Wirkstoffes behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;

b) 20 Larven von Spodoptera littoralis oder Heliothis virescens des dritten larvalen Stadiums;

c) zwei Eispiegeln von Spodoptera littoralis oder Heliothis virescens (dazu werden je 2 Blätter einer Pflanze in einem beidseitig mit Gaze verschlossenen Plexiglaszylinder eingeschlossen); zwei Eispiegel von Spodoptera oder ein Teil eines Baumwollblattes mit darauf abgelegten Eiern von Heliothis werden zu den eingeschlossenen Blättern gegeben.

Nach 4 und 5 Tagen erfolgt die Auswertung gegenüber unbehandelten Kontrollen unter Berücksichtigung folgender Kriterien:

a) Anzahl der noch lebenden Larven,

b) larvale Entwicklungs- und Häutungshemmung,

c) Frassschaden (Schabfrass und Lochfrass),

d) Schlupfrate (Anzahl der aus den Eiern geschlüpften Larven).

Verbindungen gemäss beispiel 1.2 zeigen in einer Konzentration von 400 ppm gute Gesamt-Wirksamkeit in obigem Test.

3.6 Ovizide Wirkung auf Spodoptera littoralis

Auf Filterpapier abgelegte Eier von Spodoptera littoralis werden aus dem Papier ausgeschnitten und in eine 0,05 gew.%ige Lösung des Wirkstoffes in einem Aceton/Wasser-Gemisch (1:1) getaucht. Die so

behandelten Eiablagen werden dann aus diesem Gemisch herausgenommen und bei 28° C und 60 % relativer Luftfeuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen gemäss Beispiel 1.2 zeigen gute Wirkung im obigen Test.

3.7 Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Eintrocknen der Lösung wird das Filterpapier mit den Eiern in einer Petrischale ausgelegt und bei einer Temperatur von 28° C belassen. Nach 6 Tagen wird der prozentuale Schlupf aus den behandelten Eiern bewertet.

Verbindungen gemäss Beispiel 1.2 zeigen gute Wirkung in obigem Test.

3.8 Reproduktions-Beeinflussung von Anthonomus grandis

Adulte Anthonomus grandis, die nach dem Schlupf nich älter als 24 Stunden sind, werden in Gruppen von jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.% des zu prüfenden Wirkstoffes, eingetaucht.

Wenn die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und der daraus geschlüpften Larven im Vergleich zu unbehandelten Kontrollen.

Verbindungen gemäss Beispiel 1.2 zeigen eine gute reproduktionsreduzierende Wirkung im obigen Test.

3.9 Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25° C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss Beispiel 1.2 zeigen gute Wirkung in diesem Test.

3.10 Wirkung gegen Epilachna varivestis

Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. Larvalen Stadium angesetzt. Ueber die infestierten Pflanzen wird ein Plastikzylinder gestülpt, der mit einem Kupfer-Gazedeckel abgedeckt ist. Der Versuch wird bei 28° C und 60 % relativer Luftfeuchtigkeit durchgeführt.

Nach 2 und 3 Tagen wird die Mortalität in Prozenten bestimmt. Zur Auswertung hinsichtlich allfälligem Frassschaden (Antifeeding-Effekt), Entwicklungs- und Häutungsstörungen werden die Versuchstiere während weiterer 3 Tage beobachtet.

Verbindungen gemäss Beispiel 1.2 zeigen gute Wirkung im obigen Test.

3.11 Ovizide Wirkung auf Heliothis virescens und Spodoptera littoralis

Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 400 ppm ergibt.

In diese wirkstoffhaltige Emulsion werden eintägige auf Cellophan abgelegte Eigelege von Heliothis und auf Papier abgelegte Eigelege von Spodoptera während drei Minuten eingetaucht und dann auf Rundfilter abgenutscht. Die so behandelten Gelege werden in Petrischalen ausgelegt und bei 28° C und 60 % relativer Luftfeuchtigkeit in der Dunkelheit aufbewahrt. Nach 5 bis 8 Tagen wird die Schlupfrate, d.h. die Anzahl Larven, die sich aus den behandelten Eiern entwickelt haben, gegenüber unbehandelten Kontrollansätzen bestimmt.

Verbindungen gemäss Beispiel 1.2 zeigen in obigem Test eine 80-100%ige ovizide Wirkung (Mortalität) gegen Heliothis virescens und Spodoptera littoralis.

3.12 Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Eintrocknen der Lösung werden die Eier in Petrischalen ausgelegt und bei einer Temperatur von 28° C belassen. Nach 6 Tagen wird der prozentuale Schlupf aus den behandelten Eiern bewertet und die Mortalität in Prozenten bestimmt.

Verbindungen gemäss Beispiel 1.2 zeigen gute Wirkung in obigem Test.

**Patentansprüche**

**Patentansprüche für folgende Vertragsstaaten : AT, BE, CH, DE, FR, GB, GR, IT, LI, NL**

**1.** Verbindungen der Formel I

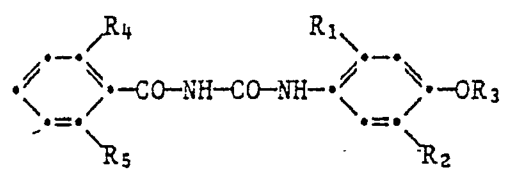

(I),

worin $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen.

**2.** Verbindungen der Formel I gemäss Anspruch 1, worin $R_1$ und $R_2$ je Fluor, Chlor oder Brom bedeuten, wobei $R_1$ und $R_2$ nicht gleichzeitig Chlor sind, $R_3$ für $-CF_2-CHF-CF_3$ $R_4$ für Wasserstoff, Fluor oder Chlor und $R_5$ für Fluor, Chlor oder Methoxy stehen.

**3.** Verbindungen der Formel I gemäss Anspruch 2, worin $R_1$ Fluor und $R_2$ Fluor, Chlor oder Brom oder $R_1$ Chlor und $R_2$ Fluor bedeuten, $R_3$ für $-CF_2-CHF-CF_3$, $R_4$ für Wasserstoff, Fluor oder Chlor und $R_5$ für Fluor, Chlor oder Methoxy stehen.

**4.** Verbindungen der Formel I gemäss Anspruch 3, worin $R_1$ Fluor und $R_2$ Fluor, Chlor oder Brom bedeuten, $R_3$ für $CF_2CHFCF_3$, $R_4$ für Wasserstoff oder Fluor und $R_5$ für Fluor, Chlor oder Methoxy stehen.

**5.** Die Verbindung gemäss Anspruch 1 der Formel

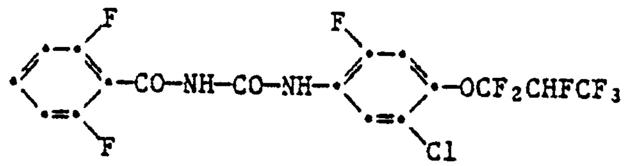 .

6. Die Verbindung gemäss Anspruch 1 der Formel

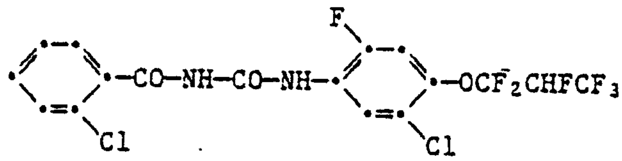 .

7. Die Verbindung gemäss Anspruch 1 der Formel

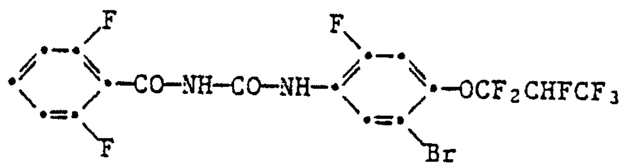 .

8. Die Verbindung gemäss Anspruch 1 der Formel

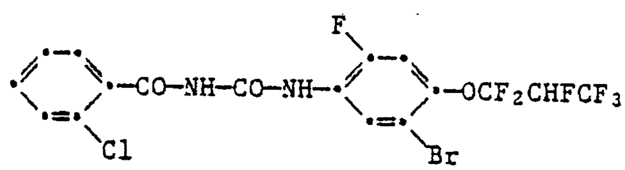 .

9. Die Verbindung gemäss Anspruch 1 der Formel

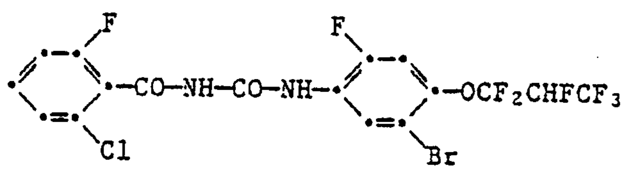 .

10. Die Verbindung gemäss Anspruch 1 der Formel

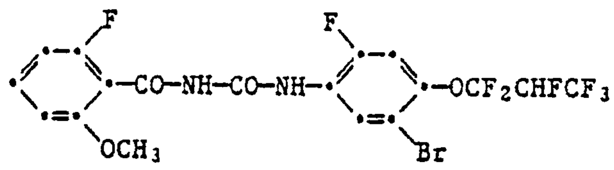

11. Die Verbindung gemäss Anspruch 1 der Formel

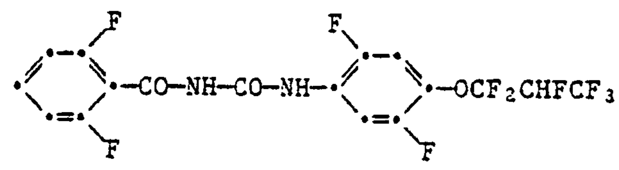

12. Die Verbindung gemäss Anspruch 1 der Formel

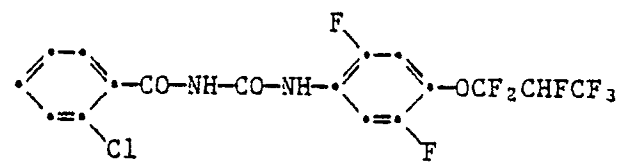

13. Die Verbindung gemäss Anspruch 1 der Formel

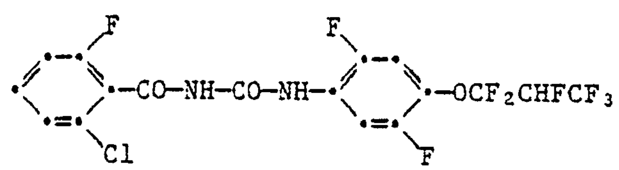

14. Die Verbindung gemäss Anspruch 1 der Formel

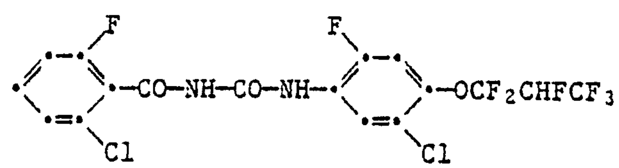

15. Die Verbindung gemäss Anspruch 1 der Formel

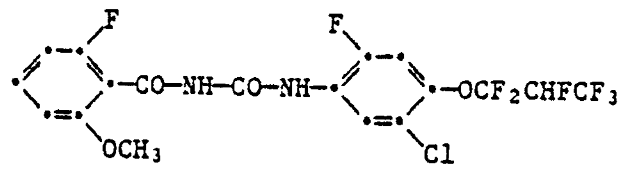

.

16. Verfahren zur Herstellung einer Verbindung der Formel I

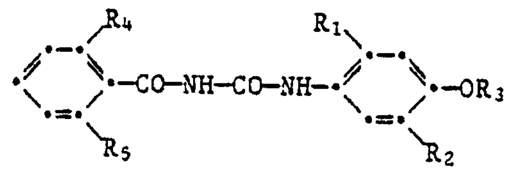

(I),

worin $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen, dadurch gekennzeichnet, dass man

a) ein Anilin der Formel II

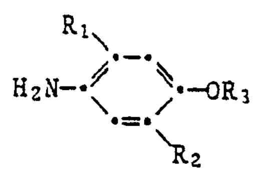

(II)

mit einem Benzoylisocyanat der Formel III

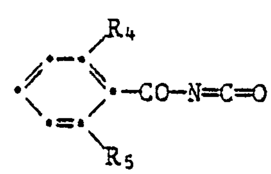

(III), oder

b) ein Isocyanat der Formel IV

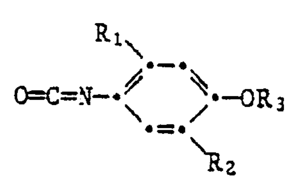

(IV)

mit einem Benzamid der Formel V

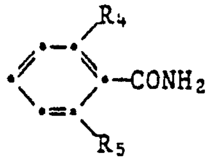

(V), oder

c) ein Anilin der Formel II mit einem Urethan der Formel VI

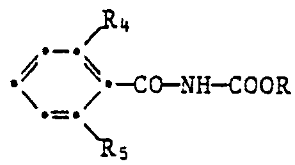

(VI)

umsetzt, wobei in den Formeln II bis VI die Symbole $R_1$, $R_2$, $R_3$, $R_4$ und $R_5$ die angegebene Bedeutung haben und R für einen gegebenenfalls halogenierten $C_1$-$C_8$-Alkylrest steht.

**17.** Verfahren gemäss Anspruch 16 zur Herstellung einer Verbindung der Formel I, dadurch gekennzeichnet, dass in der Formel I $R_1$ und $R_2$ je Fluor, Chlor oder Brom bedeuten, wobei $R_1$ und $R_2$ nicht gleichzeitig Chlor sind, $R_3$ für $-CF_2-CHF-CF_3$, $R_4$ für Wasserstoff, Fluor oder Chlor und $R_5$ für Fluor, Chlor oder Methoxy stehen.

**18.** Schädlingsbekämpfungsmittel, welches als aktive Komponente mindestens eine Verbindung der Formel I

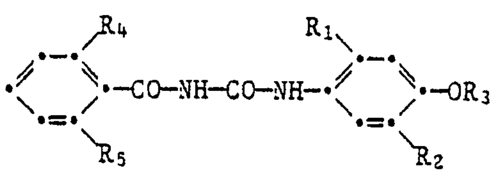

(I),

enthält, worin $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen, zusammen mit geeigneten Trägern und/oder Zuschlagstoffen.

**19.** Schädlingsbekämpfungsmittel gemäss Anspruch 18, welches als aktive Komponente mindestens eine Verbindung der Formel I enthält, worin $R_1$ und $R_2$ je Fluor, Chlor oder Brom bedeuten, wobei $R_1$ und $R_2$ nicht gleichzeitig Chlor sind, $R_3$ für $-CF_2-CHF-CF_3$, $R_4$ für Wasserstoff, Fluor oder Chlor und $R_5$ für Fluor, Chlor oder Methoxy stehen.

**20.** Schädlingsbekämpfungsmittel gemäss Anspruch 19, welches als aktive Komponente mindestens eine Verbindung der Formel I enthält, worin $R_1$ Fluor und $R_2$ Fluor, Chlor oder Brom oder $R_1$ Chlor und $R_2$ Fluor bedeuten, $R_3$ für $-CF_2-CHF-CF_3$, $R_4$ für Wasserstoff, Fluor oder Chlor und $R_5$ für Fluor, Chlor oder Methoxy stehen.

**21.** Schädlingsbekämpfungsmittel gemäss Anspruch 20, welches als aktive Komponente mindestens eine Verbindung der Formel I enthält, worin $R_1$ Fluor und $R_2$ Fluor, Chlor oder Brom bedeuten, $R_3$ für $CF_2CHFCF_3$, $R_4$ für Wasserstoff oder Fluor und $R_5$ für Fluor, Chlor oder Methoxy stehen.

**22.** Schädlingsbekämpfungsmittel gemäss einem der Ansprüche 18 bis 21, welches als aktive Komponente eine Verbindung gemäss einem der Ansprüche 5 bis 15 enthält.

**23.** Verwendung einer Verbindung der Formel I

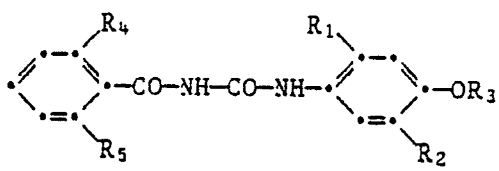

(I),

worin $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen, zur Bekämpfung von Schädlingen an Tieren und Pflanzen.

**24.** Verwendung gemäss Anspruch 23 einer Verbindung der Formel I zur Bekämpfung von Insekten und Arachniden.

**25.** Verwendung gemäss Anspruch 24 einer Verbindung der Formel I zur Bekämpfung larvaler Stadien von pflanzenschädigenden Insekten.

**26.** Verfahren zum Bekämpfen von Schädlingen an Tieren und Pflanzen, dadurch gekennzeichnet, dass man die Schädlinge in ihren verschiedenen Entwicklungsstadien mit einer Verbindung der Formel I

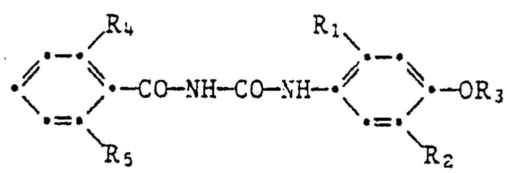

(I),

worin $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen, in Kontakt bringt.

**27.** Verbindungen der Formel II

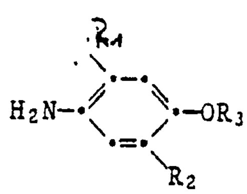

(II),

worin $R_1$ und $R_2$ je Halogen bedeuten, wobei $R_1$ und $R_2$ nicht gleichzeitig Chlor sind, und $R_3$ für $-CF_2-CHF-CF_3$ steht.

**28.** Verbindungen der Formel II gemäss Anspruch 27, worin $R_1$ und $R_2$ je Fluor, Chlor oder Brom bedeuten, wobei $R_1$ und $R_2$ nicht gleichzeitig Chlor sind, und R3 für $-CF_2-CHF-CF_3$ steht.

**29.** Verbindungen der Formel II gemäss Anspruch 28, worin $R_1$ Fluor und $R_2$ Fluor, Chlor oder Brom oder $R_1$ Chlor und $R_2$ Fluor bedeuten, und $R_3$ für $-CF_2-CHF-CF_3$ steht.

**30.** Verbindungen der Formel II gemäss Anspruch 29, worin $R_1$ Fluor und $R_2$ Fluor, Chlor oder Brom bedeuten, und $R_3$ für $CF_2CHFCF_3$ steht.

**31.** Die Verbindung gemäss Anspruch 27 der Formel

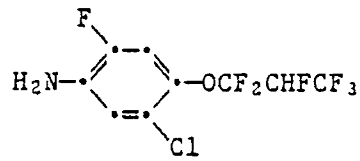

.

**32.** Die Verbindung gemäss Anspruch 27 der Formel

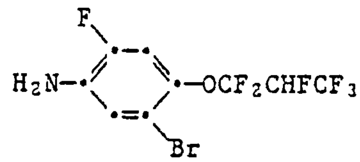

.

**33.** Die Verbindung gemäss Anspruch 27 der Formel

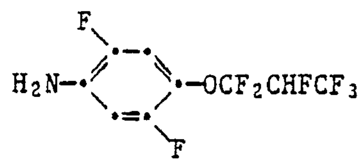

.

**Patentansprüche für folgenden Vertragsstaat : ES**

**1.** Verfahren zur Herstellung einer Verbindung der Formel I

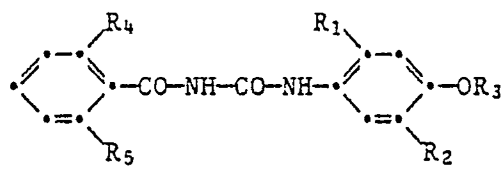

(I),

worin $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen, dadurch gekennzeichnet, dass man

a) ein Anilin der Formel II

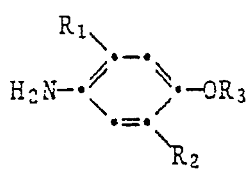

(II)

mit einem Benzoylisocyanat der Formel III

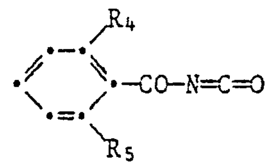

(III), oder

b) ein Isocyanat der Formel IV

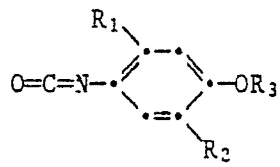

(IV)

mit einem Benzamid der Formel V

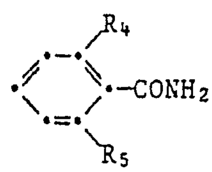

(V), oder

c) ein Anilin der Formel II mit einem Urethan der Formel VI

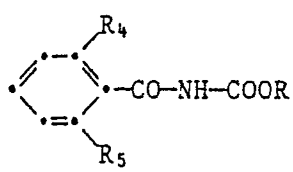

(VI)

umsetzt, wobei in den Formeln II bis VI die Symbole $R_1$, $R_2$, $R_3$, $R_4$ und $R_5$ die angegebene Bedeutung haben und R für einen gegebenenfalls halogenierten $C_1$-$C_8$-Alkylrest steht.

**2.** Verfahren gemäss Anspruch 1, worin $R_1$ und $R_2$ je Fluor, Chlor oder Brom bedeuten, wobei $R_1$ und $R_2$ nicht gleichzeitig Chlor sind, $R_3$ für $-CF_2-CHF-CF_3$ $R_4$ für Wasserstoff, Fluor oder Chlor und $R_5$ für Fluor, Chlor oder Methoxy stehen.

**3.** Verfahren gemäss Anspruch 2, worin $R_1$ Fluor und $R_2$ Fluor, Chlor oder Brom oder $R_1$ Chlor und $R_2$ Fluor bedeuten, $R_3$ für $-CF_2-CHF-CF_3$, $R_4$ für Wasserstoff, Fluor oder Chlor und $R_5$ für Fluor, Chlor oder Methoxy stehen.

**4.** Verfahren gemäss Anspruch 3, worin $R_1$ Fluor und $R_2$ Fluor, Chlor oder Brom bedeuten, $R_3$ für $CF_2CHFCF_3$, $R_4$ für Wasserstoff oder Fluor und $R_5$ für Fluor, Chlor oder Methoxy stehen.

**5.** Verfahren gemäss Anspruch 1 zur Herstellung der Verbindung der Formel

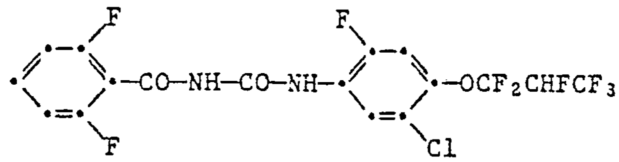
.

6. Verfahren gemäss Anspruch 1 zur Herstellung der Verbindung der Formel

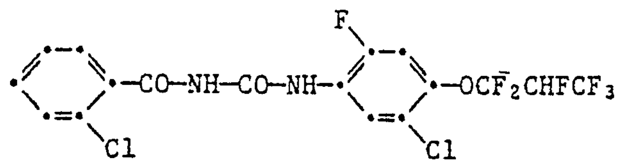
.

7. Verfahren gemäss Anspruch 1 zur Herstellung der Verbindung der Formel

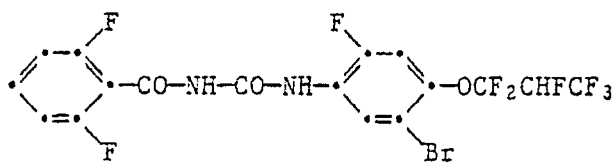
.

8. Verfahren gemäss Anspruch 1 zur Herstellung der Verbindung der Formel

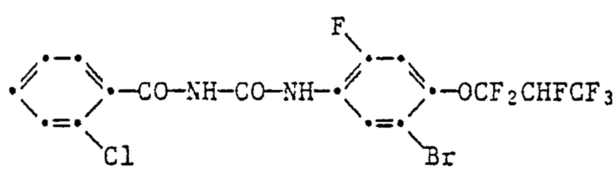
.

9. Verfahren gemäss Anspruch 1 zur Herstellung der Verbindung der Formel

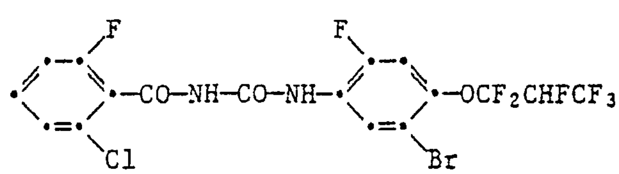
.

10. Verfahren gemäss Anspruch 1 zur Herstellung der Verbindung der Formel

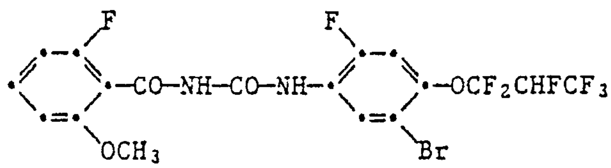

.

11. Verfahren gemäss Anspruch 1 zur Herstellung der Verbindung der Formel

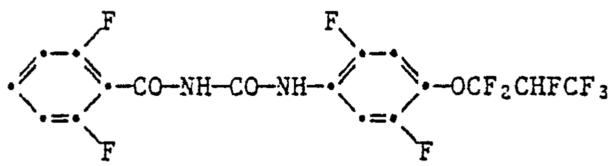

.

12. Verfahren gemäss Anspruch 1 zur Herstellung der Verbindung der Formel

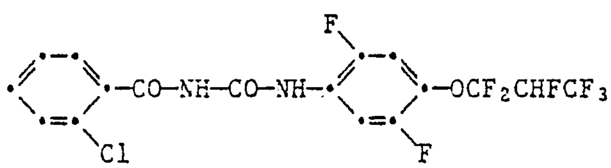

.

13. Verfahren gemäss Anspruch 1 zur Herstellung der Verbindung der Formel

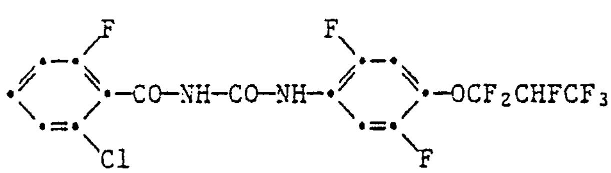

.

14. Verfahren gemäss Anspruch 1 zur Herstellung der Verbindung der Formel

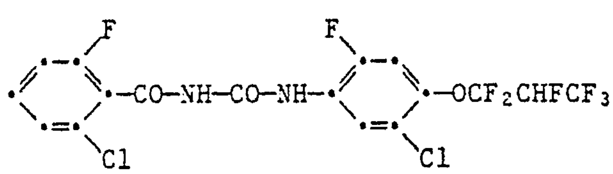

.

15. Verfahren gemäss Anspruch 1 zur Herstellung der Verbindung der Formel

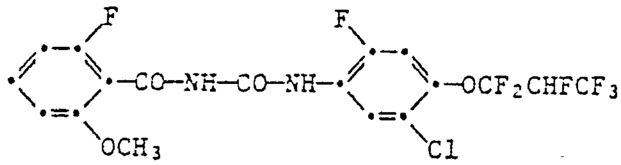

.

16. Schädlingsbekämpfungsmittel, welches als aktive Komponente mindestens eine Verbindung der Formel I

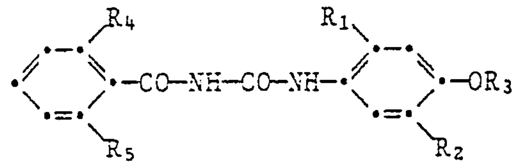

(I),

enthält, worin $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen, zusammen mit geeigneten Trägern und/oder Zuschlagstoffen.

17. Schädlingsbekämpfungsmittel gemäss Anspruch 16, welches als aktive Komponente mindestens eine Verbindung der Formel I enthält, worin $R_1$ und $R_2$ je Fluor, Chlor oder Brom bedeuten, wobei $R_1$ und $R_2$ nicht gleichzeitig Chlor sind, $R_3$ für $-CF_2-CHF-CF_3$, $R_4$ für Wasserstoff, Fluor oder Chlor und $R_5$ für Fluor, Chlor oder Methoxy stehen.

18. Schädlingsbekämpfungsmittel gemäss Anspruch 17, welches als aktive Komponente mindestens eine Verbindung der Formel I enthält, worin $R_1$ Fluor und $R_2$ Fluor, Chlor oder Brom oder $R_1$ Chlor und $R_2$ Fluor bedeuten, $R_3$ für $-CF_2-CHF-CF_3$, $R_4$ für Wasserstoff, Fluor oder Chlor und $R_5$ für Fluor, Chlor oder Methoxy stehen.

19. Schädlingsbekämpfungsmittel gemäss Anspruch 18, welches als aktive Komponente mindestens eine Verbindung der Formel I enthält, worin $R_1$ Fluor und $R_2$ Fluor, Chlor oder Brom bedeuten, $R_3$ für $CF_2CHFCF_3$, $R_4$ für Wasserstoffe oder Fluor und $R_5$ für Fluor, Chlor oder Methoxy stehen.

20. Schädlingsbekämpfungsmittel gemäss einem der Ansprüche 16 bis 19, welches als aktive Komponente eine Verbindung gemäss einem der Ansprüche 5 bis 15 enthält.

21. Verwendung einer Verbindung der Formel I

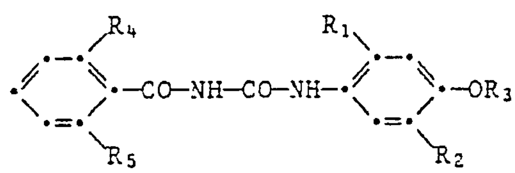

(I),

worin $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen, zur Bekämpfung von Schädlingen an Tieren und Pflanzen.

22. Verwendung gemäss Anspruch 21 einer Verbindung der Formel I zur Bekämpfung von Insekten und Arachniden.

23. Verwendung gemäss Anspruch 22 einer Verbindung der Formel I zur Bekämpfung larvaler Stadien von pflanzenschädigenden Insekten.

24. Verfahren zum Bekämpfen von Schädlingen an Tieren und Pflanzen, dadurch gekennzeichnet, dass man die Schädlinge in ihren verschiedenen Entwicklungsstadien mit einer Verbindung der Formel I

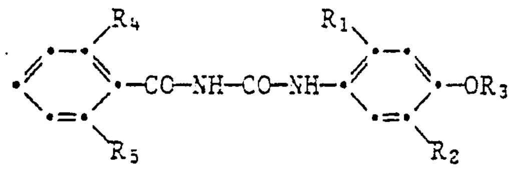 (I),

woran $R_1$ und $R_2$ je Halogen bedeuten, wobei $R_1$ und $R_2$ nicht gleichzeitig Chlor sind, $R_3$ für $-CF_2-CHF-CF_3$, $R_4$ für Wasserstoff, Halogen, Methyl, Methoxy oder Methylthio und $R_5$ für Halogen, Methyl, Methoxy oder Methylthio stehen, in Kontakt bringt.

25. Verfahren zur Hestellung der Verbindungen der Formel II

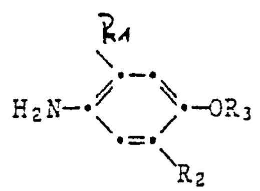 (II),

worin $R_1$ und $R_2$ je Halogen bedeuen, wobei $R_1$ und $R_2$ nicht gleichzeitig Chlor sind, und $R_3$ für $-CF_2-CHF-CF_3$ steht, dadurch gekennzeichnet, dass man eine Verbindung der Formel VII

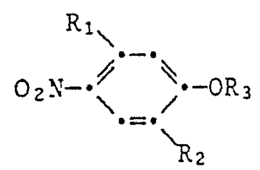 (VII)

hydriert.

26. Verfahren gemäss Anspruch 25, worin $R_1$ und $R_2$ je Fluor, Chlor oder Brom bedeuten, wobei $R_1$ und $R_2$ nicht gleichzeitig Chlor sind, und $R_3$ für $-CF_2-CHF-CF_3$ steht.

27. Verfahren gemäss Anspruch 26, worin $R_1$ Fluor und $R_2$ Fluor, Chlor oder Brom oder $R_1$ Chlor und $R_2$ Fluor bedeuten, und $R_3$ für $-CF_2-CHF-CF_3$ steht.

28. Verfahren gemäss Anspruch 26, worin $R_1$ Fluor und $R_2$ Fluor, Chlor oder Brom bedeuten, und $R_3$ für $CF_2CHFCF_3$ steht.

29. Verfahren gemäss Anspruch 26 zur Herstellung der Verbindung der Formel

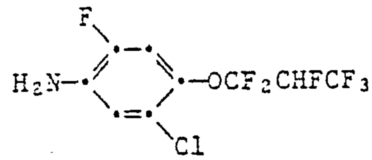

.

**30.** Verfahren gemäss Anspruch 26 zur Herstellung der Verbindung der Formel

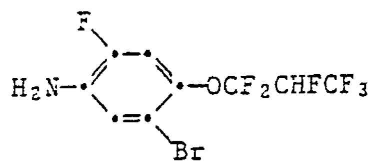

.

**31.** Verfahren gemäss Anspruch 26 zur Herstellung der Verbindung der Formel

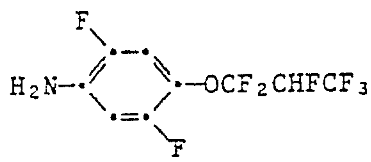

.

## Claims

**Claims for the following Contracting States : AT, BE, CH, DE, FR, GB, GR, IT, LI, NL**

**1.** A compound of formula I

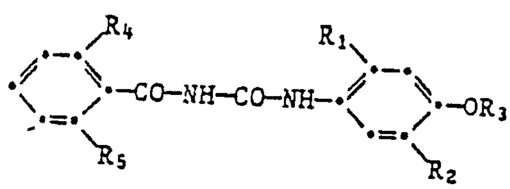

(I),

in which each of $R_1$ and $R_2$ is halogen, where $R_1$ and $R_2$ are not simultaneously chlorine, $R_3$ is $-CF_2-CHF-CF_3$, $R_4$ is hydrogen, halogen, methyl, methoxy or methylthio and $R_5$ is halogen, methyl, methoxy or methylthio.

**2.** A compound of formula I according to claim 1, in which each of $R_1$ and $R_2$ is fluorine, chlorine or bromine, where $R_1$ and $R_2$ are not simultaneously chlorine, $R_3$ is $-CF_2-CHF-CF_3$, $R_4$ is hydrogen, fluorine or chlorine and $R_5$ is fluorine, chlorine or methoxy.

**3.** A compound of formula I according to claim 2, in which $R_1$ is fluorine and $R_2$ is fluorine, chlorine or bromine or $R_1$ is chlorine and $R_2$ is fluorine, $R_3$ is $-CF_2-CHF-CF_3$, $R_4$ is hydrogen, fluorine or chlorine and $R_5$ is fluorine, chlorine or methoxy.

**4.** A compound of formula I according to claim 3, in which $R_1$ is fluorine and $R_2$ is fluorine, chlorine or bromine, $R_3$ is $CF_2CHFCF_3$, $R_4$ is hydrogen or fluorine and $R_5$ is fluorine, chlorine or methoxy.

5. The compound according to claim 1 of the formula

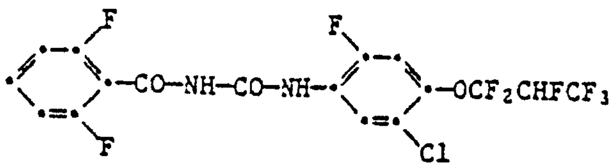
.

6. The compound according to claim 1 of the formula

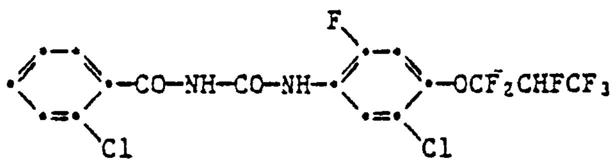
.

7. The compound according to claim 1 of the formula

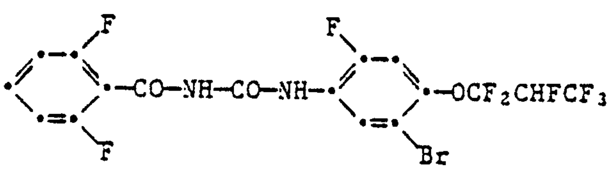
.

8. The compound according to claim 1 of the formula

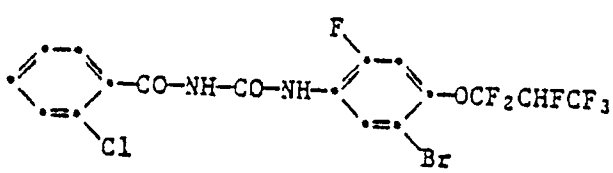
.

9. The compound according to claim 1 of the formula

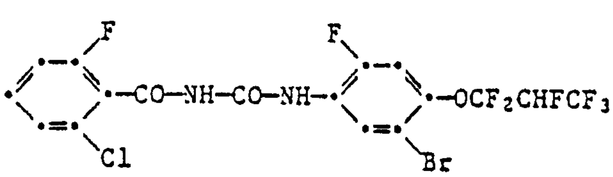
.

**10.** The compound according to claim 1 of the formula

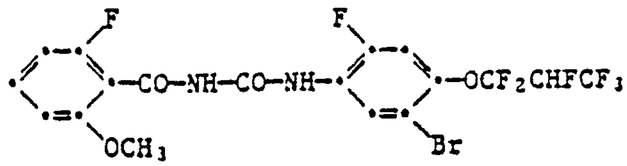 .

**11.** The compound according to claim 1 of the formula

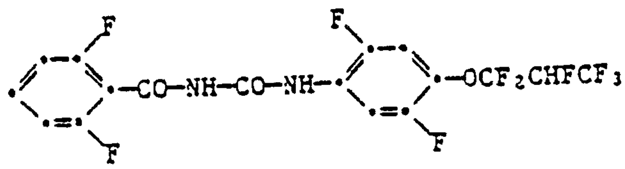 .

**12.** The compound according to claim 1 of the formula

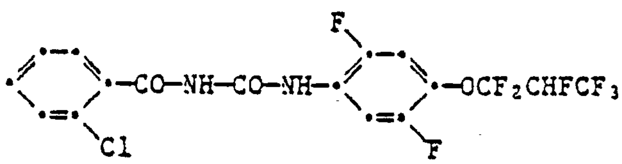 .

**13.** The compound according to claim 1 of the formula

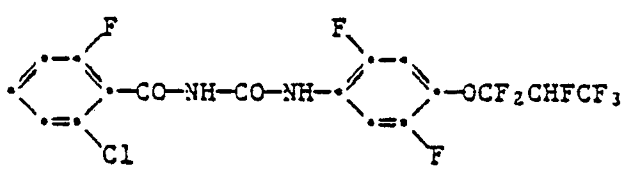 .

**14.** The compound according to claim 1 of the formula

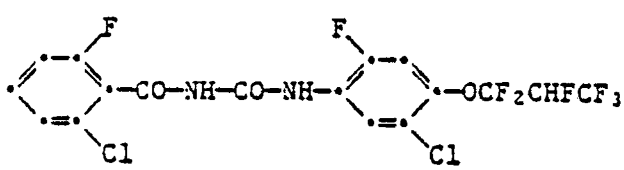 .

**15.** The compound according to claim 1 of the formula

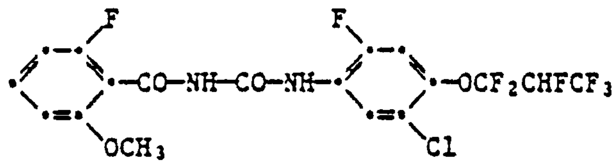

.

16. A process for the preparation of a compound of formula I

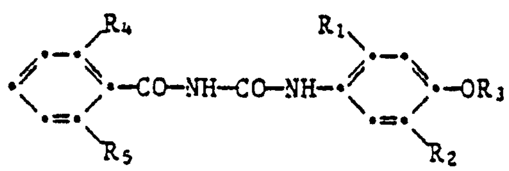

(I),

in which each of $R_1$ and $R_2$ is halogen, where $R_1$ and $R_2$ are not simultaneously chlorine, $R_3$ is $-CF_2-CHF-CF_3$, $R_4$ is hydrogen, halogen, methyl, methoxy or methylthio and $R_5$ is halogen, methyl, methoxy or methylthio, which comprises reacting

a) an aniline of formula II

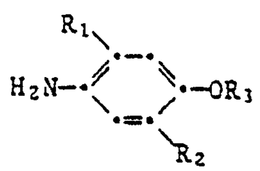

(II)

with a benzoyl isocyanate of formula III

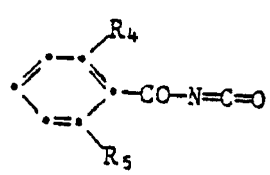

(III),

or

b) an isocyanate of formula IV

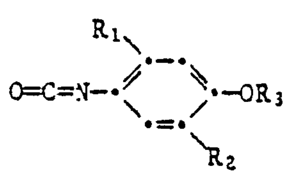

(IV)

with a benzamide of formula V

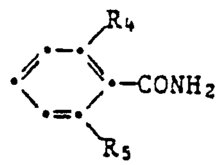

(V),

or

c) an aniline of formula II with a urethane of formula VI

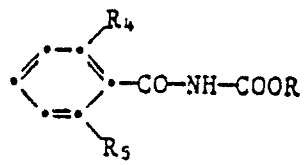

(VI)

in which formulae II to VI the symbols $R_1$, $R_2$, $R_3$, $R_4$ and $R_5$ are as defined above and R is a $C_1$-$C_8$alkyl radical which may be halogenated.

**17.** A process according to claim 16 for the preparation of a compound of formula I, wherein in formula I each of $R_1$ and $R_2$ is fluorine, chlorine or bromine, where $R_1$ and $R_2$ are not simultaneously chlorine, $R_3$ is $-CF_2-CHF-CF_3$, $R_4$ is hydrogen, fluorine or chlorine and $R_5$ is fluorine, chlorine or methoxy.

**18.** A pesticidal composition which contains, as active ingredient, at least one compound of formula I

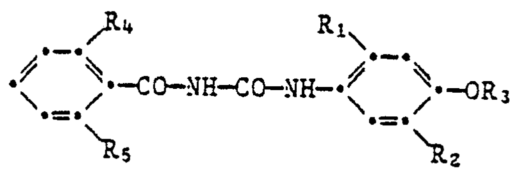

(I),

in which each of $R_1$ and $R_2$ is halogen, where $R_1$ and $R_2$ are not simultaneously chlorine, $R_3$ is $-CF_2-CHF-CF_3$, $R_4$ is hydrogen, halogen, methyl, methoxy or methylthio and $R_5$ is halogen, methyl, methoxy or methylthio, together with suitable carriers and/or adjuvants.

**19.** A pesticidal composition according to claim 18 which contains, as active ingredient, at least one compound of formula I, in which each of $R_1$ and $R_2$ is fluorine, chlorine or bromine, where $R_1$ and $R_2$ are not simultaneously chlorine, $R_3$ is $-CF_2-CHF-CF_3$, $R_4$ is hydrogen, fluorine or chlorine and $R_5$ is fluorine, chlorine or methoxy.

**20.** A pesticidal composition according to claim 19 which contains, as active ingredient, at least one compound of formula I, in which $R_1$ is fluorine and $R_2$ is fluorine, chlorine or bromine or $R_1$ is chlorine and $R_2$ is fluorine, $R_3$ is $-CF_2-CHF-CF_3$, $R_4$ is hydrogen, fluorine or chlorine and $R_5$ is fluorine, chlorine or methoxy.

**21.** A pesticidal composition according to claim 20 which contains, as active ingredient, at least one compound of formula I, in which $R_1$ is fluorine and $R_2$ is fluorine, chlorine or bromine, $R_3$ is $CF_2CHFCF_3$, $R_4$ is hydrogen or fluorine and $R_5$ is fluorine, chlorine or methoxy.

**22.** A pesticidal composition according to any one of claims 18 to 21 which contains, as active ingredient, a compound according to any one of claims 5 to 15.

23. Use of a compound of formula I

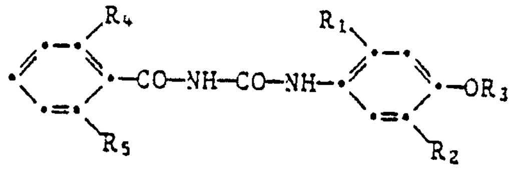

(I),

in which each of $R_1$ and $R_2$ is halogen, where $R_1$ and $R_2$ are not simultaneously chlorine, $R_3$ is $-CF_2-CHF-CF_3$, $R_4$ is hydrogen, halogen, methyl, methoxy or methylthio and $R_5$ is halogen, methyl, methoxy or methylthio, for controlling pests of animals and plants.

24. Use according to claim 23 of a compound of formula I for controlling insects and arachnids.

25. Use according to claim 24 of a compound of formula I for controlling larval stages of plant-destructive insects.

26. A method of controlling pests of animals and plants, which comprises contacting the pests in their various development stages with a compound of formula I

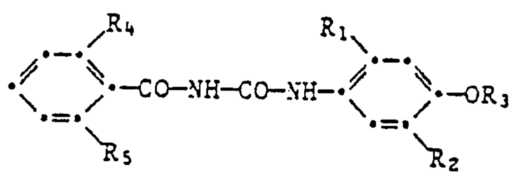

(I),

in which each of $R_1$ and $R_2$ is halogen, where $R_1$ and $R_2$ are not simultaneously chlorine, $R_3$ is $-CF_2-CHF-CF_3$, $R_4$ is hydrogen, halogen, methyl, methoxy or methylthio and $R_5$ is halogen, methyl, methoxy or methylthio.

27. A compound of formula II

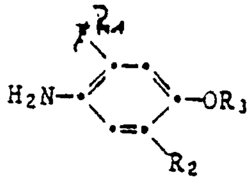

(II),

in which each of $R_1$ and $R_2$ is halogen, where $R_1$ and $R_2$ are not simultaneously chlorine, and $R_3$ is $-CF_2-CHF-CF_3$.

28. A compound of formula II according to claim 27, in which each of $R_1$ and $R_2$ is fluorine, chlorine or bromine, where $R_1$ and $R_2$ are not simultaneously chlorine, and $R_3$ is $-CF_2-CHF-CF_3$.

29. A compound of formula II according to claim 28, in which $R_1$ is fluorine and $R_2$ is fluorine, chlorine or bromine or $R_1$ is chlorine and $R_2$ is fluorine and $R_3$ is $-CF_2-CHF-CF_3$.

30. A compound of formula II according to claim 29, in which $R_1$ is fluorine and $R_2$ is fluorine, chlorine or bromine and $R_3$ is $CF_2CHFCF_3$.

**31.** The compound according to claim 27 of the formula

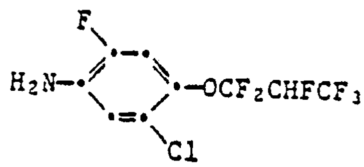

.

**32.** The compound according to claim 27 of the formula

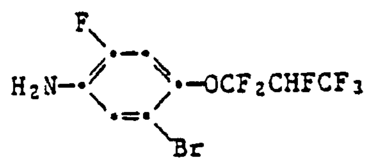

.

**33.** The compound according to claim 27 of the formula

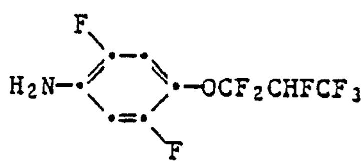

.

**Claims for the following Contracting State : ES**

**1.** A process for the preparation of a compound of formula I

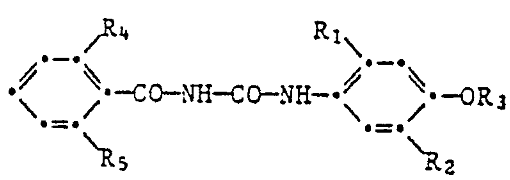

(I),

in which each of $R_1$ and $R_2$ is halogen, where $R_1$ and $R_2$ are not simultaneously chlorine, $R_3$ is -$CF_2$-CHF-$CF_3$, $R_4$ is hydrogen, halogen, methyl, methoxy or methylthio and $R_5$ is halogen, methyl, methoxy or methylthio, which comprises reacting

a) an aniline of formula II

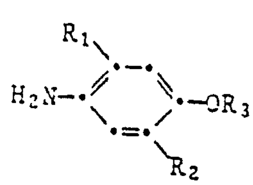

(II)

with a benzoyl isocyanate of formula III

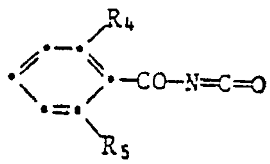 (III),

or

b) an isocyanate of formula IV

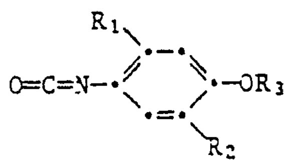 (IV)

with a benzamide of formula V

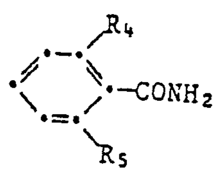 (V),

or

c) an aniline of formula II with a urethane of formula VI

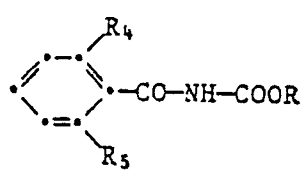 (VI)

in which formulae II to VI the symbols $R_1$, $R_2$, $R_3$, $R_4$ and $R_5$ are as defined above and R is a $C_1$-$C_8$alkyl radical which may be halogenated.

2. A process according to claim 1, in which each of $R_1$ and $R_2$ is fluorine, chlorine or bromine, where $R_1$ and $R_2$ are not simultaneously chlorine, $R_3$ is $-CF_2-CHF-CF_3$, $R_4$ is hydrogen, fluorine or chlorine and $R_5$ is fluorine, chlorine or methoxy.

3. A process according to claim 2, in which $R_1$ is fluorine and $R_2$ is fluorine, chlorine or bromine or $R_1$ is chlorine and $R_2$ is fluorine, $R_3$ is $-CF_2-CHF-CF_3$, $R_4$ is hydrogen, fluorine or chlorine and $R_5$ is fluorine, chlorine or methoxy.

4. A process according to claim 3, in which $R_1$ is fluorine and $R_2$ is fluorine, chlorine or bromine, $R_3$ is $CF_2CHFCF_3$, $R_4$ is hydrogen or fluorine and $R_5$ is fluorine, chlorine or methoxy.

5. A process according to claim 1 for the preparation of the compound of the formula

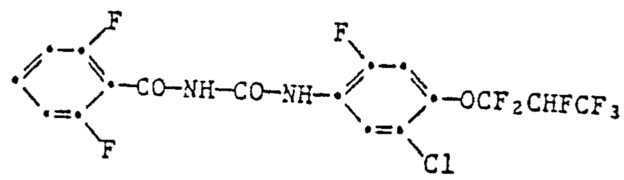.

6. A process according to claim 1 for the preparation of the compound of the formula

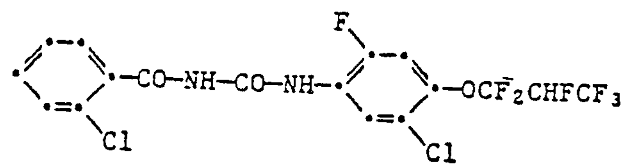.

7. A process according to claim 1 for the preparation of the compound of the formula

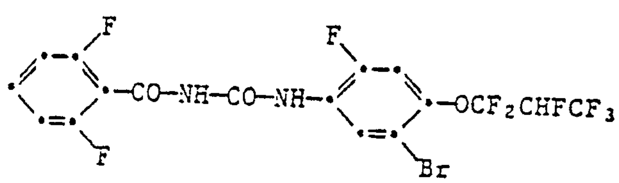.

8. A process according to claim 1 for the preparation of the compound of the formula

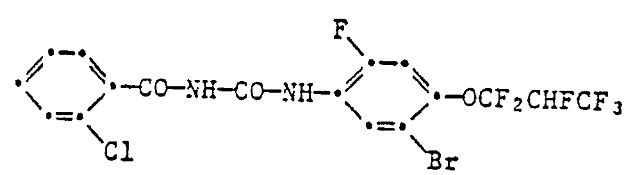.

9. A process according to claim 1 for the preparation of the compound of the formula

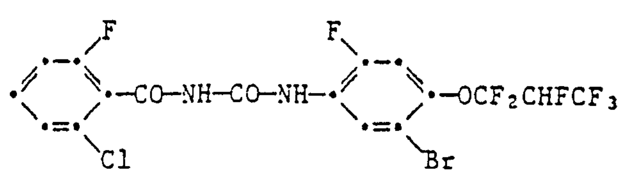.

10. A process according to claim 1 for the preparation of the compound of the formula

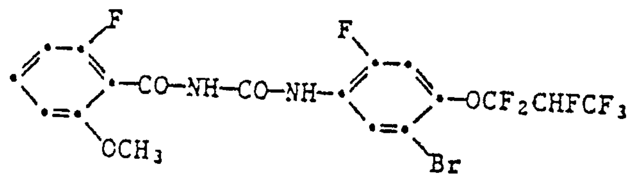

.

11. A process according to claim 1 for the preparation of the compound of the formula

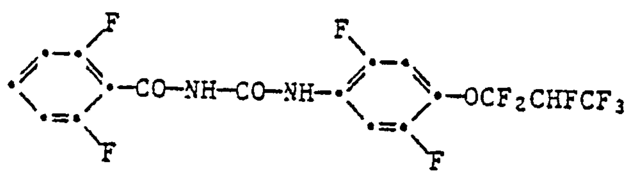

.

12. A process according to claim 1 for the preparation of the compound of the formula

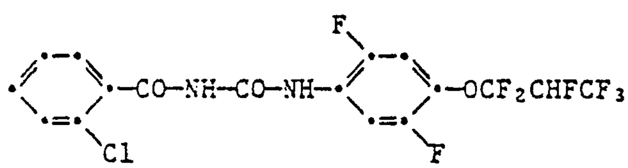

.

13. A process according to claim 1 for the preparation of the compound of the formula

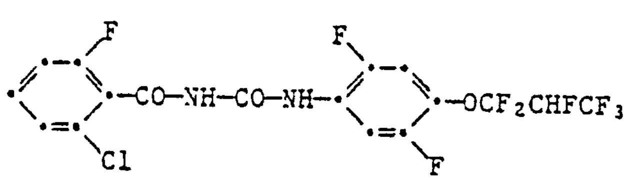

.

14. A process according to claim 1 for the preparation of the compound of the formula

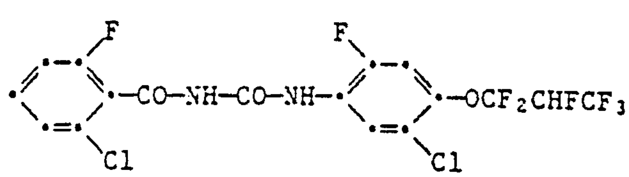

.

15. A process according to claim 1 for the preparation of the compound of the formula

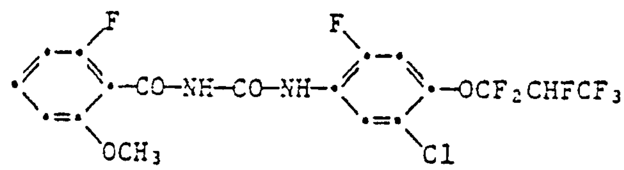

**16.** A pesticidal composition which contains, as active ingredient, at least one compound of formula I

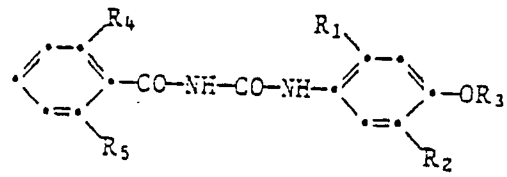 (I),

in which each of $R_1$ and $R_2$ is halogen, where $R_1$ and $R_2$ are not simultaneously chlorine, $R_3$ is $-CF_2-CHF-CF_3$, $R_4$ is hydrogen, halogen, methyl, methoxy or methylthio and $R_5$ is halogen, methyl, methoxy or methylthio, together with suitable carriers and/or adjuvants.

**17.** A pesticidal composition according to claim 16 which contains, as active ingredient, at least one compound of formula I, in which each of $R_1$ and $R_2$ is fluorine, chlorine or bromine, where $R_1$ and $R_2$ are not simultaneously chlorine, $R_3$ is $-CF_2-CHF-CF_3$, $R_4$ is hydrogen, fluorine or chlorine and $R_5$ is fluorine, chlorine or methoxy.

**18.** A pesticidal composition according to claim 17 which contains, as active ingredient, at least one compound of formula I, in which $R_1$ is fluorine and $R_2$ is fluorine, chlorine or bromine or $R_1$ is chlorine and $R_2$ is fluorine, $R_3$ is $-CF_2-CHF-CF_3$, $R_4$ is hydrogen, fluorine or chlorine and $R_5$ is fluorine, chlorine or methoxy.

**19.** A pesticidal composition according to claim 18 which contains, as active ingredient, at least one compound of formula I, in which $R_1$ is fluorine and $R_2$ is fluorine, chlorine or bromine, $R_3$ is $CF_2CHFCF_3$, $R_4$ is hydrogen or fluorine and $R_5$ is fluorine, chlorine or methoxy.

**20.** A pesticidal composition according to any one of claims 16 to 19 which contains, as active ingredient, a compound according to any one of claims 5 to 15.

**21.** Use of a compound of formula I

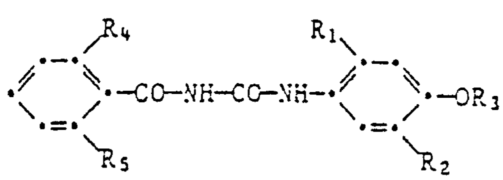 (I),

in which each of $R_1$ and $R_2$ is halogen, where $R_1$ and $R_2$ are not simultaneously chlorine, $R_3$ is $-CF_2-CHF-CF_3$, $R_4$ is hydrogen, halogen, methyl, methoxy or methylthio and $R_5$ is halogen, methyl, methoxy or methylthio, for controlling pests of animals and plants.

**22.** Use according to claim 21 of a compound of formula I for controlling insects and arachnids.

23. Use according to claim 22 of a compound of formula I for controlling larval stages of plant-destructive insects.

24. A method of controlling pests of animals and plants, which comprises contacting the pests in their various development stages with a compound of formula I

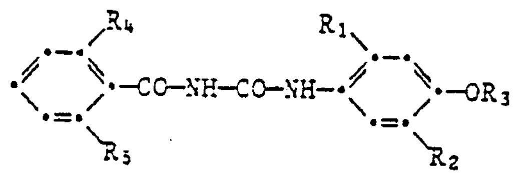

(I),

in which each of $R_1$ and $R_2$ is halogen, where $R_1$ and $R_2$ are not simultaneously chlorine, $R_3$ is $-CF_2-CHF-CF_3$, $R_4$ is hydrogen, halogen, methyl, methoxy or methylthio and $R_5$ is halogen, methyl, methoxy or methylthio.

25. A process for the preparation of a compound of formula II

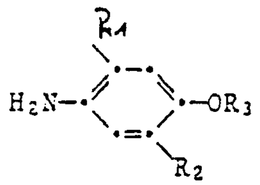

(II),

in which each of $R_1$ and $R_2$ is halogen, where $R_1$ and $R_2$ are not simultaneously chlorine, and $R_3$ is $-CF_2-CHF-CF_3$, which comprises hydrogenating a compound of formula VII

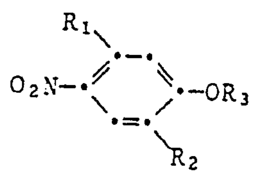

(VII)

26. A process according to claim 25, in which each of $R_1$ and $R_2$ is fluorine, chlorine or bromine, where $R_1$ and $R_2$ are not simultaneously chlorine, and $R_3$ is $-CF_2-CHF-CF_3$.

27. A process according to claim 26, in which $R_1$ is fluorine and $R_2$ is fluorine, chlorine or bromine or $R_1$ is chlorine and $R_2$ is fluorine and $R_3$ is $-CF_2-CHF-CF_3$.

28. A process according to claim 26, in which $R_1$ is fluorine and $R_2$ is fluorine, chlorine or bromine and $R_3$ is $CF_2CHFCF_3$.

29. A process according to claim 26 for the preparation of the compound of the formula

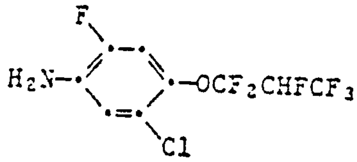

.

30. A process according to claim 26 for the preparation of the compound of the formula

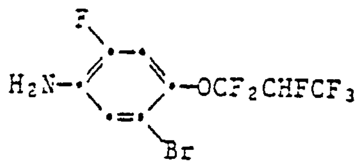

.

31. A process according to claim 26 for the preparation of the compound of the formula

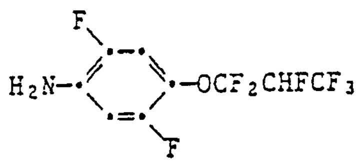

.

**Revendications**

**Revendications pour les Etats contractants suivants : AT, BE, CH, DE, FR, GB, GR, IT, LI, NL**

1. Composés de formule I

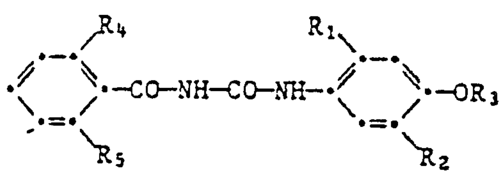

(I),

dans laquelle $R_1$ et $R_2$ représentent chacun un halogène mais ne peuvent représenter tous deux simultanément le chlore, $R_3$ représente $-CF_2-CHF-CF_3$, $R_4$ représente l'hydrogène, un halogène, un groupe méthyle, méthoxy ou méthylthio, et $R^5$ représente un halogène, un groupe méthyle, méthoxy ou méthylthio.

2. Composés de formule I de la revendication 1, dans laquelle $R_1$ et $R_2$ représente chacun le fluor, le chlore ou le brome mais ne peuvent représenter tous deux simultanément le chlore, $R_3$ représente $-CF_2-CHF-CF_3$, $R_4$ représente l'hydrogène, le fluor ou le chlore, et $R_5$ le fluor, le chlore ou un groupe méthoxy.

3. Composés de formule I de la revendication 2, dans laquelle $R_1$ représente le fluor et $R_2$ représente le fluor, le chlore ou le brome, ou bien $R_1$ représente le chlore et $R_2$ le fluor, $R_3$ représente $-CF_2-CHF-CF_3$, $R_4$ représente l'hydrogène, le fluor ou le chlore et $R_5$ le fluor, le chlore ou un groupe méthoxy.

4. Composés de formule I de la revendication 3, dans laquelle $R_1$ représente le fluor et $R_2$ le fluor, le

chlore ou le brome, $R_3$ représente $-CF_2-CHF-CF_3$, $R_4$ représente l'hydrogène ou le fluor et $R_5$ le fluor, le chlore ou un groupe méthoxy.

5. Le composé de la revendication 1, de formule

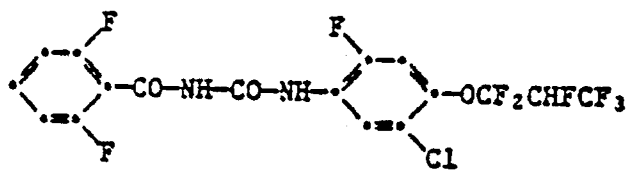

6. Le composé de la revendication 1, de formule

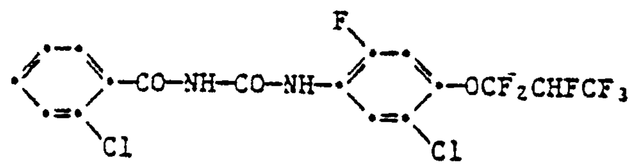

7. Le composé de la revendication 1, de formule

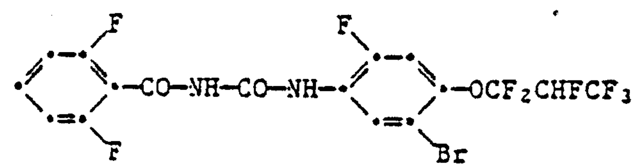

8. Le composé de la revendication 1, de formule

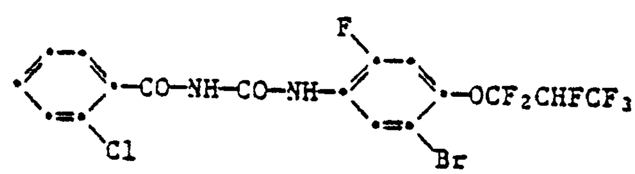

9. Le composé de la revendication 1, de formule

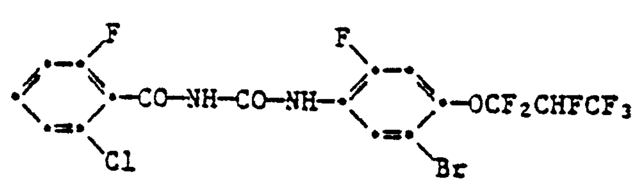

10. Le composé de la revendication 1, de formule

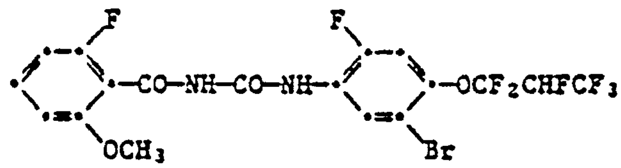

**11.** Le compose de la revendication 1, de formule

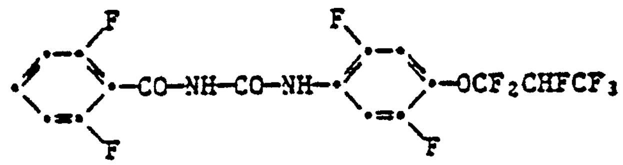

**12.** Le composé de la revendication 1, de formule

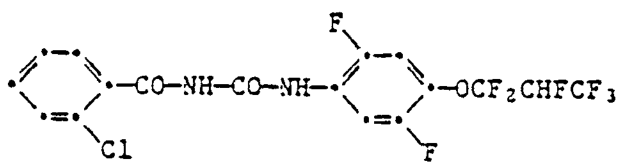

**13.** Le composé de la revendication 1, de formule

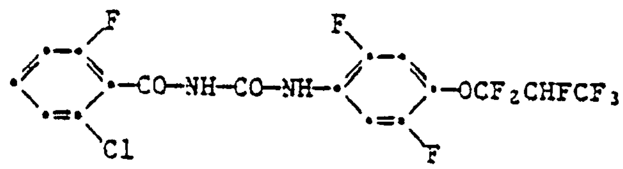

**14.** Le composé de la revendication 1, de formule

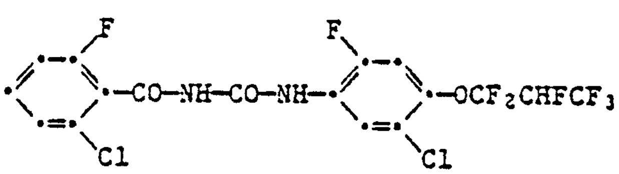

**15.** Le composé de la revendication 1, de formule

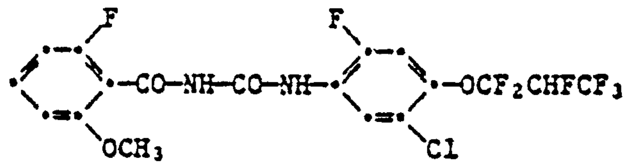

**16.** Procédé de préparation d'un composé de formule I

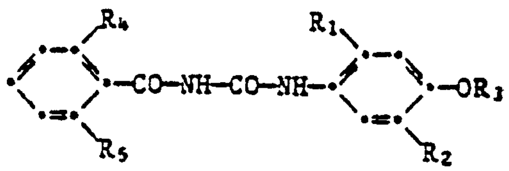

(I),

dans laquelle $R_1$ et $R_2$ représentent chacun un halogène mais ne peuvent représenter tous deux le chlore, $R_3$ représente $-CF_2-CHF-CF_3$, $R_4$ représente l'hydrogène, un halogène, un groupe méthyle, méthoxy ou méthylthio, et $R_5$ un halogène, un groupe méthyle, méthoxy ou méthylthio, caractérisé en ce que :

a) on fait réagir une aniline de formule II

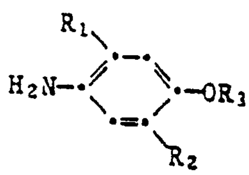

(II)

avec un isocyanate de benzoyle de formule III

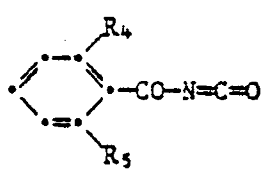

(III),

ou bien

b) on fait réagir un isocyanate de formule IV

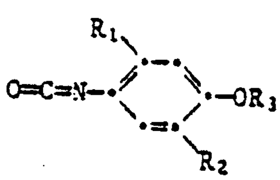

(IV)

avec un benzamide de formule V

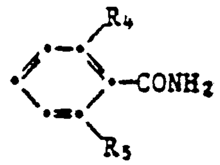

(V),

ou bien

c) on fait réagir une aniline de formule II avec un uréthanne de formule VI

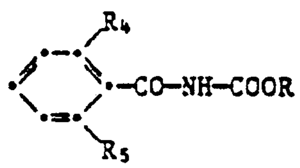

(VI)

les symboles $R_1$, $R_2$, $R_3$, $R_4$ et $R_5$ ayant dans les formules II à VI les significations indiquées ci-dessus et R représentant un groupe alkyle en C 1-C 8 éventuellement halogéné.

**17.** Procédé selon la revendication 16, pour préparer un composé de formule I, caractérisé en ce que, dans la formule I, $R_1$ et $R_2$ représentent chacun le fluor, le chlore ou le brome mais ne peuvent représenter tous deux simultanément le chlore, $R_3$ représente $-CF_2-CHF-CF_3$, $R_4$ représente l'hydrogène, le fluor ou le chlore et $R_5$ le fluor, le chlore ou un groupe méthoxy.

**18.** Produit pesticide contenant au moins un composant actif consistant en un composé de formule I

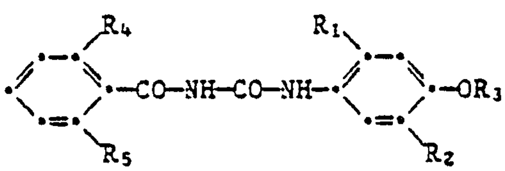

(I),

dans laquelle $R_1$ et $R_2$ représentent chacun un halogène mais ne peuvent représenter tous deux simultanément le chlore, $R_3$ représente $-CF_2-CHF-CF_3$, $R_4$ représente l'hydrogène, un halogène, un groupe méthyle, méthoxy ou méthylthio et $R_5$ un halogène, un groupe méthyle, méthoxy ou méthylthio, avec des véhicules et/ou des additifs appropriés.

**19.** Produit pesticide selon la revendication 18, contenant au moins un composant actif consistant en un composé de formule I dans laquelle $R_1$ et $R_2$ représentent chacun le fluor, le chlore ou le brome mais ne peuvent représenter tous deux simultanément le chlore, $R_3$ représente $-CF_2-CHF-CF_3$, $R_4$ représente l'hydrogène, le fluor ou le chlore et $R_5$ le fluor, le chlore ou un groupe méthoxy.

**20.** Produit pesticide selon la revendication 19, contenant au moins un composant actif consistant en un composé de formule I dans laquelle $R_1$ représente le fluor et $R_2$ le fluor, le chlore ou le brome, ou bien $R_1$ représente le chlore et $R_2$ le fluor, $R_3$ représente $-CF_2-CHF-CF_3$, $R_4$ représente l'hydrogène, le fluor ou le chlore et $R_5$ le fluor, le chlore ou un groupe méthoxy.

**21.** Produit pesticide selon la revendication 20, contenant au moins un composant actif consistant en un composé de formule I dans laquelle $R_1$ représente le fluor et $R_2$ le fluor, le chlore ou le brome, $R_3$ représente $-CF_2-CHF-CF_3$, $R_4$ représente l'hydrogène ou le fluor et $R_5$ le fluor, le chlore ou un groupe méthoxy.

**22.** Produit pesticide selon l'une des revendications 18 à 21, contenant en tant que composant actif un composé selon l'une des revendications 5 à 15.

**23.** Utilisation d'un composé de formule I

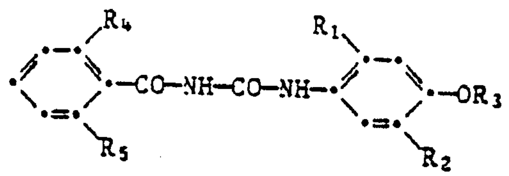

(I),

dans laquelle $R_1$ et $R_2$ représentent chacun un halogène mais ne peuvent représenter tous deux simultanément le chlore, $R_3$ représente $-CF_2-CHF-CF_3$, $R_4$ représente l'hydrogène, un halogène, un groupe méthyle, méthoxy ou méthylthio et $R_5$ représente un halogène, un groupe méthyle, méthoxy ou méthylthio, pour la lutte contre les parasites des animaux et des végétaux.

**24.** Utilisation selon la revendication 23 d'un composé de formule I pour la lutte contre les insectes et les arachnides.

**25.** Utilisation selon la revendication 24 d'un composé de formule I pour la lutte contre les stades larvaires des insectes nuisibles pour les végétaux.

**26.** Procédé pour combattre les parasites des animaux et des végétaux, caractérisé en ce que l'on met les parasites, dans leurs divers stades de développement, en contact avec un composé de formule I

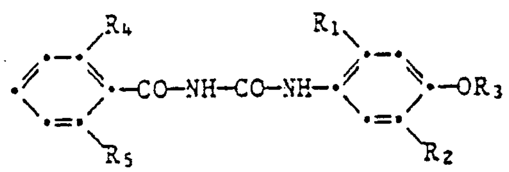

(I),

dans laquelle $R_1$ et $R_2$ représentent chacun un halogène mais ne peuvent représenter tous deux simultanément le chlore, $R_3$ représente $-CF_2-CHF-CF_3$, $R_4$ représente l'hydrogène, un halogène, un groupe méthyle, méthoxy ou méthylthio et $R_5$ représente un halogène, un groupe méthyle, méthoxy ou méthylthio.

**27.** Composés de formule II

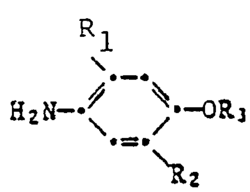

(II),

dans laquelle $R_1$ et $R_2$ représentent chacun un halogène mais ne peuvent représenter tous deux simultanément le chlore, $R_3$ représente $-CF_2-CHF-CF_3$.

**28.** Composés de formule II de la revendication 27, dans laquelle $R_1$ et $R_2$ représentent chacun le fluor, le chlore ou le brome mais ne peuvent représenter tous deux simultanément le chlore et $R_3$ représente $-CF_2-CHF-CF_3$.

**29.** Composés de formule II de la revendication 28, dans laquelle $R_1$ représente le fluor et $R_2$ le fluor, le chlore ou le brome ou bien $R_1$ représente le chlore et $R_2$ le fluor et $R_3$ représente $-CF_2-CHF-CF_3$.

**30.** Composés de formule II de la revendication 29, dans laquelle $R_1$ représente le fluor et $R_2$ le fluor, le

chlore ou le brome, et $R_3$ représente $-CF_2-CHF-CF_3$.

**31.** Le composé de la revendication 27, de formule

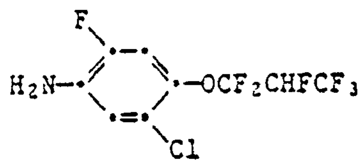

**32.** Le composé de la revendication 27, de formule

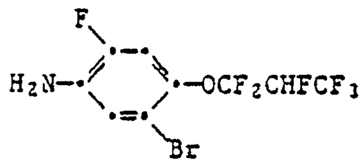

**33.** Le composé de la revendication 27, de formule

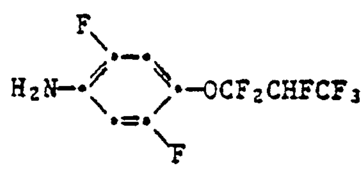

**Revendications pour l'Etat contractant suivant : ES**

**1.** Procédé de préparation d'un composé de formule I

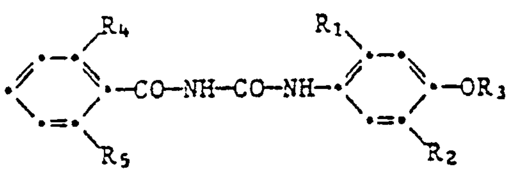

(I),

dans laquelle $R_1$ et $R_2$ représentent chacun un halogène mais ne peuvent représenter tous deux simultanément le chlore, $R_3$ représente $-CF_2-CHF-CF_3$, $R_4$ représente l'hydrogène, un groupe méthyle, méthoxy ou méthylthio, et $R_5$ un halogène, un groupe méthyle, méthoxy ou méthylthio, caractérisé en ce que :

a) on fait réagir une aniline de formule II

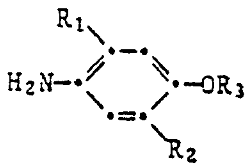

(II)

avec un isocyanate de benzoyle de formule III

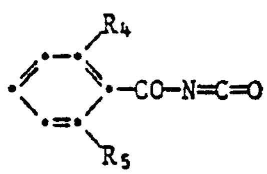

(III),

ou bien

b) on fait réagir un isocyanate de formule IV

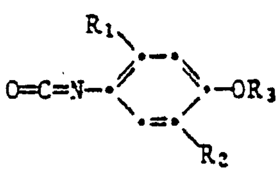

(IV)

avec un benzamide de formule V

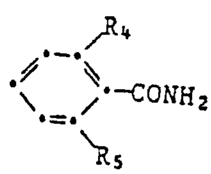

(V),

ou bien

c) on fait réagir une aniline de formule II avec un uréthanne de formule VI

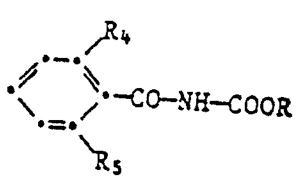

(VI),

les symboles $R_1$, $R_2$, $R_3$, $R_4$ et $R_5$ ayant dans les formules II à VI les significations indiquées ci-dessus et R représentant un groupe alkyle en C 1-C 8 éventuellement halogéné.

**2.** Procédé selon la revendication 1, dans lequel $R_1$ et $R_2$ représentent chacun le fluor, le chlore ou le brome mais ne peuvent représenter tous deux simultanément le chlore, $R_3$ représente $-CF_2-CHF-CF_3$, $R_4$ représente l'hydrogène, le fluor ou le chlore et $R_5$ représente le fluor, le chlore ou un groupe méthoxy.

3. Procédé selon la revendication 2, dans lequel $R_1$ représente le fluor et $R_2$ le fluor, le chlore ou le brome, ou bien $R_1$ représente le chlore et $R_2$ le fluor, $R_3$ représente $-CF_2-CHF-CF_3$, $R_4$ représente l'hydrogène, le fluor ou le chlore et $R_5$ le fluor, le chlore, ou un groupe méthoxy.

4. Procédé selon la revendication 3, dans lequel $R_1$ représente le fluor et $R_2$ le fluor, le chlore ou le brome, $R_3$ représente $CF_2CHFCF_3$, $R_4$ représente l'hydrogène ou le fluor, et $R_5$ le fluor, le chlore ou un groupe méthoxy.

5. Procédé selon la revendication 1, pour la préparation du composé de formule

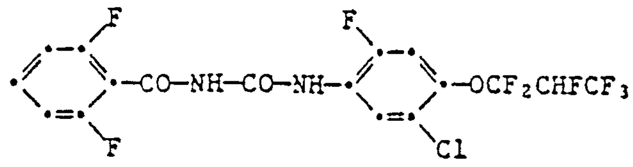

6. Procédé selon la revendication 1, pour la préparation du composé de formule

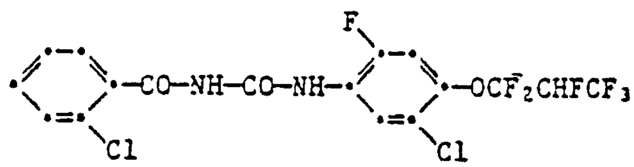

7. Procédé selon la revendication 1, pour la préparation du composé de formule

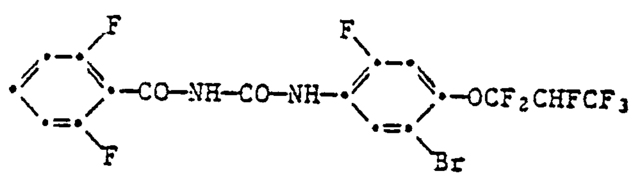

8. Procédé selon la revendication 1, pour la préparation du composé de formule

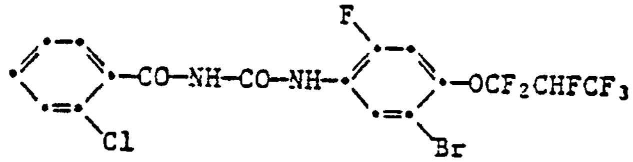

9. Procédé selon la revendication 1, pour la préparation du composé de formule

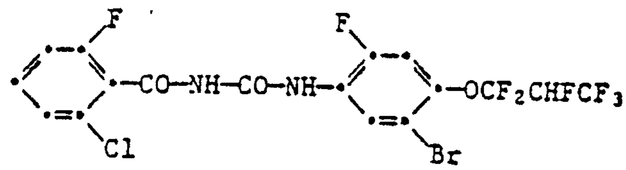

10. Procédé selon la revendication 1, pour la préparation du composé de formule

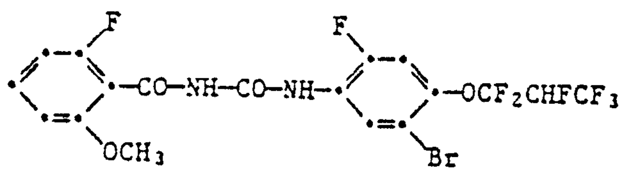

11. Procédé selon la revendication 1, pour la préparation du composé de formule

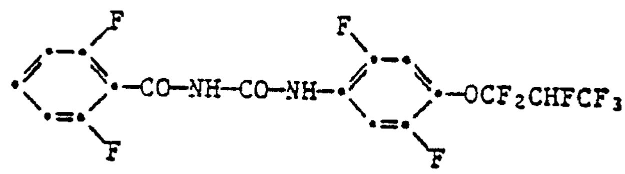

12. Procédé selon la revendication 1, pour la préparation du composé de formule

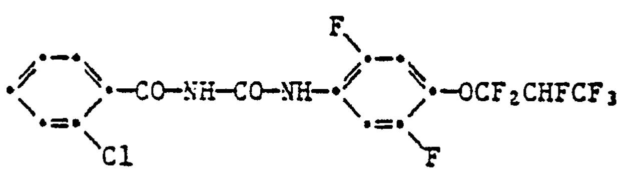

13. Procédé selon la revendication 1, pour la préparation du composé de formule

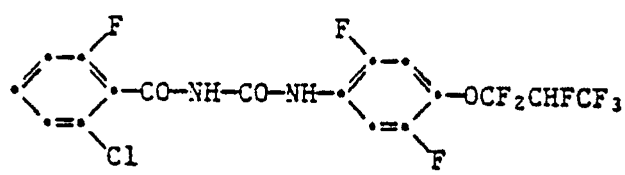

14. Procédé selon la revendication 1, pour la préparation du composé de formule

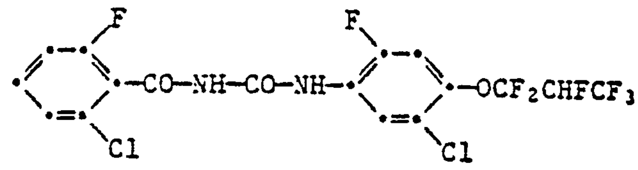

**15.** Procédé selon la revendication 1, pour la préparation du composé de formule

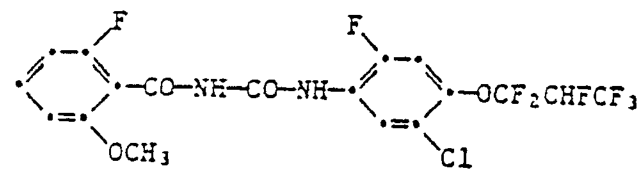

**16.** Produit pesticide contenant au moins un composant actif consistant en un composé de formule I

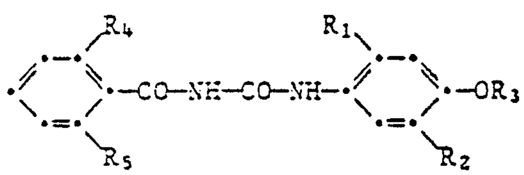

(I),

dans laquelle $R_1$ et $R_2$ représentent chacun un halogène mais ne peuvent représenter tous deux simultanément le chlore, $R_3$ représente $-CF_2-CHF-CF_3$, $R_4$ représente l'hydrogène, un halogène, un groupe méthyle, méthoxy ou méthylthio et $R_5$ un halogène, un groupe méthyle, méthoxy ou méthylthio, avec des véhicules et/ou additifs appropriés.

**17.** Produit pesticide selon la revendication 16, contenant au moins un composant actif consistant en un composé de formule I dans laquelle $R_1$ et $R_2$ représentent chacun le fluor, le chlore ou le brome mais ne peuvent représenter tous deux simultanément le chlore, $R_3$ représente $-CF_2-CHF-CF_3$, $R_4$ représente l'hydrogène, le fluor ou le chlore et $R_5$ le fluor, le chlore ou un groupe méthoxy.

**18.** Produit pesticide selon la revendication 17, contenant au moins un composant actif consistant en un composé de formule I dans laquelle $R_1$ représente le fluor et $R_2$ le fluor, le chlore ou le brome, ou bien $R_1$ représente le chlore et $R_2$ le fluor, $R_3$ représente $-CF_2-CHF-CF_3$, $R_4$ représente l'hydrogène, le fluor ou le chlore, et $R_5$ le fluor, le chlore ou un groupe méthoxy.

**19.** Produit pesticide selon la revendication 18, contenant au moins un composant actif consistant en un composé de formule I dans laquelle $R_1$ représente le fluor et $R_2$ le fluor, le chlore ou le brome, $R_3$ représente $CF_2CHFCF_3$, $R_4$ représente l'hydrogène ou le fluor, et $R_5$ le fluor, le chlore ou un groupe méthoxy.

**20.** Produit pesticide selon l'une des revendications 16 à 19, contenant un composé selon l'une des revendications 5 à 15 en tant que composant actif.

**21.** Utilisation d'un composé de formule I

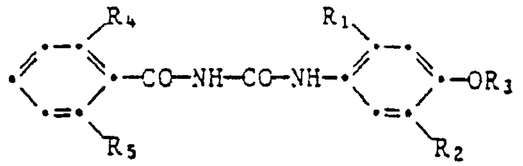

(I),

dans laquelle $R_1$ et $R_2$ représentent chacun un halogène mais ne peuvent représenter tous deux simultanément le chlore, $R_3$ représente $-CF_2-CHF-CF_3$, $R_4$ représente l'hydrogène, un halogène ou un groupe méthyle, méthoxy ou méthylthio, et $R_5$ un halogène, un groupe méthyle, méthoxy ou méthylthio, pour la lutte contre les parasites des animaux et des végétaux.

**22.** Utilisation selon la revendication 21 d'un composé de formule I pour la lutte contre les insectes et les arachnides.

**23.** Utilisation selon la revendication 22 d'un composé de formule I pour la lutte contre les stades larvaires des insectes nuisibles pour les végétaux.

**24.** Procédé pour combattre les parasites des animaux et des végétaux, caractérisé en ce que l'on met les parasites, dans leurs divers stades de développement, en contact avec un composé de formule I

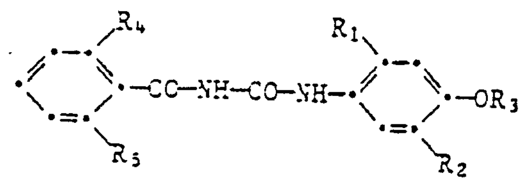

(I),

dans laquelle $R_1$ et $R_2$ représentent chacun un halogène mais ne peuvent représenter tous deux simultanément le chlore, $R_3$ représente $-CF_2-CHF-CF_3$, $R_4$ représente l'hydrogène, un halogène, un groupe méthyle, méthoxy ou méthylthio, et $R_5$ un halogène, un groupe méthyle, méthoxy ou méthylthio.

**25.** Procédé de préparation des composés de formule II

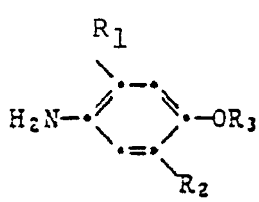

(II),

dans laquelle $R_1$ et $R_2$ représentent chacun un halogène mais ne peuvent représenter tous deux simultanément le chlore, $R_3$ représente $-CF_2-CHF-CF_3$, caractérisé en ce que l'on hydrogène un composé de formule VII

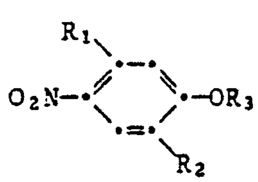

(VII)

26. Procédé selon la revendication 25, dans laquelle $R_1$ et $R_2$ représentent chacun le fluor, le chlore ou le brome mais ne peuvent représenter tous deux simultanément le chlore, et $R_3$ représente $-CF_2-CHF-CF_3$.

27. Procédé selon la revendication 26, dans lequel $R_1$ représente le fluor et $R_2$ le fluor, le chlore ou le brome, ou bien $R_1$ représente le chlore et $R_2$ le fluor, et $R_3$ représente $-CF_2-CHF-CF_3$.

28. Procédé selon la revendication 26, dans lequel $R_1$ représente le fluor et $R_2$ le fluor, le chlore ou le brome, et $R_3$ représente $CF_2CHFCF_3$.

29. Procédé selon la revendication 26, pour la préparation du composé de formule

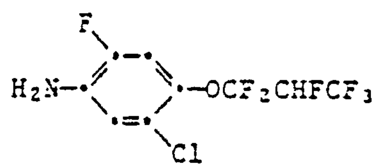

30. Procédé selon la revendication 26, pour la préparation du composé de formule

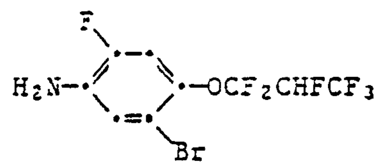

31. Procédé selon la revendication 26, pour la préparation du composé de formule